# EUROPEAN PATENT SPECIFICATION

(11) **EP 4 735 899 B1**
(45) Date of publication and mention of the grant of the patent: **11.11.2026**
(21) Application number: 25701707.9
(22) Date of filing: 16.01.2025
(51) Int. Cl.: G01Q 60/32, C12M 1/42, G01N 33/483, G01N 33/487, G01Q 60/36, G01Q 60/42, G01N 15/10, G01N 21/47

(54) **METHOD OF STIMULATING A LIVING HUMAN OR ANIMAL CELL USING A CHARACTERISTIC SIGNAL OF THE CELL AND RELATED APPLIANCE**
VERFAHREN ZUR STIMULATION EINER LEBENDEN MENSCHLICHEN ODER TIERISCHEN ZELLE UNTER VERWENDUNG EINES CHARAKTERISTISCHEN SIGNALS DER ZELLE UND ZUGEHÖRIGES GERÄT
PROCÉDÉ DE STIMULATION D'UNE CELLULE HUMAINE OU ANIMALE VIVANTE À L'AIDE D'UN SIGNAL CARACTÉRISTIQUE DE LA CELLULE ET APPAREIL ASSOCIÉ

(30) Priority: 16.01.2024 IT 202400000693
(43) Date of publication of application: 06.05.2026
(73) Proprietor: Eldor Lab S.r.l., 20124 Milano (MI) (IT)
(72) Inventor: VENTURA, Carlo, 40121 Bologna BO (IT)
(74) Representative: Barbaro, Gaetano
(86) International application number: PCT/IB2025/050466
(87) International publication number: WO 2025/153985

(56) References cited:
- US-A1- 2020 181 576
- KROL A. YU. ET AL: "Local mechanical oscillations of the cell surface within the range 0.2-30 Hz", EUROPEAN BIOPHYSICS JOURNAL, vol. 19, no. 2, 1 November 1990 (1990-11-01), DE, pages 93 - 99, XP093185049, ISSN: 0175-7571, DOI: 10.1007/BF00185092
- PELLING ANDREW E. ET AL: "Local Nanomechanical Motion of the Cell Wall of Saccharomyces cerevisiae", SCIENCE, vol. 305, no. 5687, 20 August 2004 (2004-08-20), pages 1147 - 1150, XP093184871

## Description

### TECHNICAL FIELD

This disclosure relates to analysis of biological patterns and more in detail a method of stimulating a living human or animal cell either in vivo or in vitro extracted from a human or animal body using a characteristic signal of the cell, and a related appliance.

### BACKGROUND

Increasing evidence show that biological patterns are fashioned not only by chemical, but even physical signaling, including mechanical waves, electric patterning and gradients, as well as electromagnetic radiations, which also include light [1]. Such physical energies are sensed and released by somatic and stem cells, and are currently gaining consideration as a part of a morphogenetic code underlying the causal relationship between the establishing of defined domains at the molecular level, the emergence of supramolecular structures, the timely unfolding of subcellular and cellular shapes, up to the appearance of the large-scale anatomy of tissue and organs and the entire individual specification. When thinking at ourselves as a part of the oscillatory nature of the Universe, we became aware that physical forces are essential in the orchestration of living organisms, as it is shown by: (i) the physical dynamics of molecular folding, (ii) the progressive deciphering of mechanical and electromagnetic patterning essential in the establishment of nano-architectonics using suprainteractions, and (iii) the biomolecular recognition and signal propagation afforded through dynamics of molecular synchronization and swarming [1].

At the physical level, all biological processes entail a form of vibration. By the aid of sophisticated devices and methods, including atomic force microscopy (AFM) [2-5], scanning tunneling microscopy (STM) [6,7], terahertz near-field microscopy (THz-NFM) [8], and hyperspectral imaging (HSI) [9-11] we are now dissecting and deciphering specific vibrational patterning at subcellular and cellular level. Thus, a single peptide molecule appears as helix-loop-helix repeats. These are intrinsically oscillatory domains, as the helices behave as a spring (oscillator), with the loops acting as inter-oscillator linkers. These springs are electrically polarized, including both the positively-charged amino groups (such as those in Lysine and Arginine), and the negatively-charged carboxyl groups (such as those in Aspartate and Glutamate), as well as the backbone dipole interactions in the formation of secondary and super-secondary protein structures. Therefore, the helices in a signaling molecule not only behave as mechanical oscillators, but acquire the state of an electromechanical actuator, with electro-mechanical interacting potential with other similar helix-loop-helix modules from a multitude of signaling players.

As a result, the mechanical oscillation of such cell signaling actuators is also capable of generating an electric field, with radiation characteristics. Compounding the multi-level features of complexity ensuing from such view, a consistent number of signaling molecules are now found to unfold their vibrational features into the capability of absorbing and emitting light within defined domain spectra, therefore being deemed as "chromophores". The list of such chromophore molecules is constantly increasing over time, now including flavins, flavoproteins and cytochromes [12-16], such as those responsible for the generation of reactive oxygen species (ROS) and nitric oxide [15,17-19], which act as essential pleiotropic players in cellular dynamics. There is now mounting evidence that essential signaling paths in both somatic and stem cells are controlled by opsins, a group of cis-retinal dependent G protein-coupled receptors, encompassing members of the family of transient receptor potential cation channels (TRPs) [14,20,21]. TRPs encompass multiple superfamily members which are selectively operated by specific light wavelengths, playing a pivotal role in cellular decisions [22-26], as photoentrainment and cellular circadian rhythms [26].

Intriguingly, melanopsin (Opn4), a non-image-forming opsin, has been shown to afford a physiological role in the modulation of blood vessel function, particularly in the context of photoinduced vascular relaxation (photorelaxation) [27]. Opn4 is expressed in blood vessels. Force-tension myography provided evidence that vessels from Opn4^{-/-}mice lacked photorelaxation, which was also abrogated by an Opn4-specific inhibitor. The observed photorelaxation was wavelength-specific, and did not involve endothelial, nitric oxide-, carbon monoxide-, or cytochrome p450-derived vasoactive prostanoid signaling, but was associated with vascular hyperpolarization, and was also soluble guanylyl cyclase- and phosphodiesterase 6-dependent [27].

The presence of Opn4 in blood vessels has also been confirmed in pulmonary arterial smooth muscle cells and pulmonary arteries with a crucial role of light in inducing Opn4-mediated vasorelaxation and increase in blood tissue perfusion [28,29]. These findings, considered together with the observation that light radiation regulated tail artery vasoreactivity ex vivo and enhanced tail blood flow in vivo [27], indicate a major role of light radiation as a major conductor of vascular biodynamics, and suggest that endogenous, vessel embedded chromophores may be the potential target for unprecedented therapy in vascular diseases. Overall, mechanical and light patterning coalesce within the same biological substrates, whether such substrates are single signaling peptides (i.e. chromophores) or their supramolecular assemblies, including, but not limited to, tubulin dimers, microtubuli, or a vessel wall, since mechanical waves will hardly separate from the generation of electric oscillations and light sensing/emission [6,27]. Conversely, the exposure of such signaling actuators to an electromagnetic field or light has been shown to produce mechanical oscillations [6]. Thus, the intrinsic physical features of signaling molecules embed both mechanical and electromagnetic (light) patterns, as a whole, inseparable ensemble of physical forces.

Hereinafter, we will therefore refer to vibrations as an "inclusive term", representing the oneness of morphogenetic physical cues emerging from, and being sensed by the cellular circuitries of signaling molecules. These physical features are expressed as, and respond to, precise vibrational profiles. In particular, cytoskeletal and nucleoskeletal elements, as well as the intercellular connecting net of tunneling nanotubes, produce concerted electromechanical waves, merging mechanical vibrations and mechano-sensing with the generation of electric and electromagnetic oscillations, harboring the features of connectedness, long-range force radiation and even multilevel memory-switching properties.

STM analysis has documented the formation of these memory states, involving vibrational (mechanical and electromagnetic) modalities controlling the protein arrangement symmetry associated with the conducting state embedded within the nanowire structure of cellular microtubuli, which makes a single microtubule creating a random-access memory analogue of the flash memory switch used in a computer chip [30]. Microtubuli themselves share similarities with the ordered arrangement of photoactive molecules with large transition dipole moments, and the analysis of tryptophan molecules, the amino acid building block of microtubules with the largest transition dipole strength, revealed that their arrangement within the microtubular configuration exhibits a superradiant lowest exciton state, which represents an excitation fully extended on the chromophore lattice [31]. Theoretical formulations have suggested that such superradiant state emerges due to supertransfer coupling between the lowest exciton states of smaller blocks of the microtubule. In this theoretical modeling, the velocity of photoexcitation spreading was boosted by the supertransfer effect, when compared to the expected velocity from the strength of the nearest-neighbor coupling between microtubular tryptophan moieties [31]. On the whole, these modeling hints suggest that microtubule and the cytoskeleton itself may act as a bioelectronic circuit providing an informational flow emerging from a multitude of self-organizing vibrations, ensuing and propagating as both short- and long-range/lived pattern of mechanical and electromagnetic (including light) waves.

Synchronization and swarming patterns within such bioelectronic circuit, extend to the oscillatory network of signaling molecules and molecular motors [32,33]. Such collective behavior uses evolving forms of nanoarchitectonics and supra(molecular)interactions, including the generation of vortices coalescing into a lattice structure, as building blocks of biological information processes [32,33].

The possibility that such signaling actuators play a role in the emergence of a morphogenetic code is strongly suggested by the finding that the biological effects elicited by cellular exposure to mechanical or electromagnetic (including light) energies are remarkably more accentuated, and recruit a wider repertoire of cellular responses and fate decisions, when these physical energies are delivered as pulse-modulated rather than continuous waves [34-38]. We have shown that embryonic and human adult stem cells can be subjected to specific differentiation fates by the aid of asymmetrically conveyed electromagnetic fields [39,40], and that extremely low-frequency magnetic fields remarkably change gene transcription in rat adult cardiomyocytes [41], to elicit high efficiency cardiogenesis in mouse embryonic stem cells [42]. We have also shown that asymmetrically conveyed electromagnetic fields prime differentiation of non-stem human somatic cells, including skin fibroblasts, into cardiac, neural, and skeletal muscle cells, a lineage repertoire in which somatic cells would never otherwise manifest [43].

Moreover, we proved that the same electromagnetic energy could reverse human stem cell senescence in vitro by activating both telomerase-dependent and -independent pathways [44,45], and reprogram tumor cells of neural origin into dopaminergic neurons [46].

Nevertheless, definitive evidence is lacking that:
- Precise, specific signatures of the differentiation patterns of stem cells along specific phenotypes (i.e., but not limited to, cardiac, and vascular lineages), can be achieved at the nanomechanical level.
- Said nanomechanical signatures can be released in the form of mechanical, as well as light patterns to targeted undifferentiated (stem) cells to orchestrate their differentiation into specific phenotypes (i.e. myocardial, and vascular lineages).
- Said nanomechanical signatures can be released in the form of light patterns to target chromophores (i.e. melanopsins) embedded within the blood vessels to elicit photorelaxation and enhance tissue perfusion, optimizing tissue health, or promoting tissue rescue and survival to hostile environments (i.e., but not limited to, hypoxia, ischemia, oxidative stress, deranged tissue vascularization, pathological aging).
- Said nanomechanical signatures can be released in the form of light patterns to enhance tissue microcirculation and wound healing, and afford reverse remodeling of tissue scars, therefore reversing aging processes which occur within the context of impaired blood supply and tissue fibrosis.

US2020/0181576 discloses methods of acquiring cellular and tissue vibrational patterning to identify signatures, and more in particular it relates to the delivery of such signatures to human adult stem cells or human adult somatic cells to induce specific differentiation processes and promote survival to hostile environmental conditions.

The article by Krol A. Yu. et al. "Local mechanical oscillations of the cell surface within the range 0.2-30Hz", European Biophysics Journal, vol. 19, no. 2, 1 November 1990, pages 93-99, discloses an optical device for detecting mechanical oscillations of a cell surface.

The article by Pelling Andrew E. et al. "Local nanomechanical motion of the cell wall of saccharomyces cerevisiae", Science, vol. 305, no. 5687, 20 August 2004, pages 1147-1150, discloses the use of an atomic force microscope (AFM) to measure oscillation frequencies of cells. The article is focused on detecting periodic movements (i.e. oscillations) in the range from 0.8 to 1.6kHz.

### SUMMARY

To solve at least in part the above limitations, the present disclosure provides a method according to claim 1 of stimulating a living human or animal cell either in vivo or in vitro extracted from a human or animal body, by generating a characteristic signal - i.e. the "signature" - of a living human or animal cell extracted from a human or animal body; generating a mechanical stimulation or a modulated light stimulation by means of a piezo-electric or light actuator controlled according to data of the characteristic signal of said living human or animal cell, wherein the modulated light stimulation is generated by modulating with the characteristic signal an intensity of a light beam absorbable by the living human or animal cell; applying said mechanical or light stimulation to said living human or animal cell. This "signature" is obtained throughout the following operations:
placing the extracted living cell into a test solution;
sensing a vibration of a superficial portion of an external surface of the extracted living cell;
generating a transduced time-varying signal corresponding to the sensed vibration. In other words, the time-varying signal is determined by the sense vibration and is generated according thereto;
low-pass filtering values of the transduced time-varying signal in the frequency range below 20Hz, generating data of the characteristic signal as a low-pass filtered replica of the values of the transduced time-varying signal.

According to one aspect, this "signature" may be generated using an AFM by placing its sensing tip in contact with the external surface of the extracted living cell.

According to an alternative, this "signature" may be generated using an illuminating laser configured to illuminate the external surface of the cell and an optical sensor configured to sense light scattered by the external surface of the cell.

Preferably, the characteristic signal - i.e. the "signature" - is generated as a low-pass filtered replica in the frequency range below 10Hz, preferably below 8Hz.

It is also disclosed an appliance according to claim 5 for stimulating a living human or animal cell, either in vivo or in vitro, extracted from a human or animal body.

Other embodiments are defined in the annexed claims.

### BRIEF DESCRIPTION OF THE DRAWINGS

Figure 1 shows A) image acquired by the optical microscope on which the AFM head is mounted showing the cantilever positioned on top of a beating cardiomyocyte. The red spot shows the laser of the cantilever deflection measurement system; B) signal obtained from the lateral torsion of the cantilever. Several cycles are reported. The y-axis reports the voltage signal obtained from the photodetector.
Figures 2A, 2B, show the schemes of the development of the Uniaxial Stretcher.
Figure 3 shows the scheme for the Isotropic Stretcher development.
Figure 4 shows the picture of the Uniaxial Stretcher used to convey the mechanical strain signal to cells in vitro, inside an on-top stage incubator connected to an inverted optical microscope.
Figures 5A, 5B show the pictures of the Uniaxial Stretcher inside a standard CO₂ cell culture incubator.
Figure 6 shows pictures of the Isotropic Stretcher on the stage of an inverted optical microscope (A), and details of the inside driving dynamics (B).
Figure 7 shows PDMS (Polydimethylsiloxane) supports used to perform cyclic stretching experiments: A) support for the uniaxial stretching device; B) support for the isotropic stretching device; C) support for the isotropic stretching device.
Figure 8 illustrates strategies for the reconstruction of the signal to be conveyed by the stretching devices; A) strategy based on the low-pass filtering where the frequency cutoff is established considering the Fast Fourier Transform (FFT) of the AFM-derived signal; B) strategy based on the reconstruction exploiting the first 8 peaks of the FFT spectrum.
Figure 9 shows discretization of the cardiac signal performed in order to convey the signal by stepper motors with constant step length.
Figure 10 illustrates pictures of the Light-Emitting Actuator Inducing Cardiac Differentiation by the Delivery of an AFM-acquired Cardiogenic Signature through a Mechanical Actuator.
Figure 11 illustrates profile of gene expression patterning in human induced pluripotent stem cells (hiPS) exposed to AFM-acquired cardiogenic signature delivered by Isotropic Stretcher as described above. The levels of each individual mRNA was assessed by software Bio-Rad Maestro at the end of Real Time-PCR. n=4, p<0.05. Samples with duplicates that differed by Ct >0.5 points were excluded from the analysis (mean ± SEM).
Figure 12 illustrates expression of GATA6 (red) and TRA-1-60 (green) in hiPS cultured under control, unexposed conditions (A), or exposed to AFM-acquired cardiogenic signature by the aid of the Isotropic Stretcher described herein (B). Samples were fixed in PFA 4% and stained following classical immunocytology protocols. Cells were analyzed after the exposure to the physical stimulation and cardiogenic differentiation.
Figure 13 shows protein expression of α-sarcomeric actinin in hiPS exposed to AFM-acquired cardiogenic signature delivered by Isotropic Stretcher (T) compared to control unexposed cells (C). Proteins were analyzed after the exposure to the physical stimulation and cardiogenic differentiation as described above (mean ± SEM, of 4 individual experiments).
Figure 14 illustrates expression of troponin T (TnT, red) in hiPS exposed to AFM-acquired cardiogenic signature delivered by Isotropic Stretcher for 4 days and differentiated into cardiomyocytes (B), as compared to control unexposed cells (A).
Figure 15 illustrates in vitro vasculogenesis from human umbilical vein endothelial cells (HUVECs) that had been exposed in the absence (A), or presence (B) of AFM-acquired cardiogenic signature by the aid of the Light-Emitting Actuator described herein. C, capillary length; D, capillary branching (arbitrary units); (mean ± SEM, of 4 individual experiments).
Figure 16 illustrates the expression of specific antigens involved in angiogenesis of HUVECs that had been exposed in the absence (Control), or presence (Exposed) of AFM-acquired cardiogenic signature by the aid of the Light-Emitting Actuator described herein, at 24 hours (T1), 48 hours (T2), and 72 hours (T3). A, endothelial Nitric Oxide Synthase, eNOS; B, Phosphatidylinositol 4,5-bisphosphate or PtdIns(4,5)P2,Pip₂; C, inducible Nitric Oxide Synthase, iNOS; (fluorescence is calculated as arbitrary units); (mean ± SEM, of 4 individual experiments).
Figure 17 depicts representative images of scratch closure at time 0 and 22h in human mesenchymal stem cells (hMSCs) that had been pretreated for 3h in the absence (A,C) or presence (B,D) of AFM-acquired cardiogenic signature delivered by the Light-Emitting Actuator described herein. Histogram E represents the percentage of scratch closure at 22h in control unexposed (Control) or exposed cells (Exposed) (mean ± SEM, of 4 individual experiments).
Figure 18 illustrates in vitro vasculogenesis form co-culture of HUVECs and hMSCs, that had been exposed in the absence (Control), or presence (Exposed) of AFM-acquired cardiogenic signature by the aid of the Light-Emitting Actuator described herein. Quantification of increased expression of specific proteins involved in vasculogenesis. A, CD31, B, vascular endothelial (VE)-cadherin, (fluorescence is calculated as arbitrary units); C, number of competent segments; D, number of intersections; E, percentage of total area covered by competent segments (mean ± SEM, of 4 individual experiments).
Figure 19 illustrates the effect of 1- or 3-h pre-treatment of hMSCs with AFM-acquired cardiogenic signature delivered by a Light-Emitting Actuator on the gene expression of β-catenin (A), and phospho-p38 MAP Kinase (B), as compared to unexposed control cells (C).
Figure 20 illustrates the effect of a 24-h treatment of human cardiac fibroblasts (hCFs) with AFM-acquired cardiogenic signature delivered by Isotropic Stretcher on the expression of fibronectin. A) unexposed control; B) signature exposed hCFs; C) hCFs exposed to a scrambled signature. Each panel is representative of a 2-mm² area.
Figure 21 illustrates the effect of a 24-h treatment of hCFs with AFM-acquired cardiogenic signature delivered by Isotropic Stretcher on the expression of osteopontin. A) unexposed control; B) signature exposed hCFs; C) hCFs exposed to a scrambled signature. Each panel is representative of a 2-mm² area.
Figure 22 illustrates a differential accumulation of hyaluronic acid (HA) in control unexposed hCFs, and hCFs that had been exposed to the AFM-acquired cardiogenic signature delivered by Isotropic Stretcher. A), HA levels in the supernatant; B), cell-associated HA expression. White bars, control unexposed cells. Black bars, signature-exposed hCFs. Hyaluronan was assessed by HA-binding protein-based test kit (Corgenix Medical Corp., Broomfield, CO, USA); (mean ± SEM, of 4 individual experiments).
Figures 23 to 26 show illustrative embodiments of wearable bands, as well as a helmet, and a bite, according to the present disclosure for stimulating cells in vivo.
Figure 27 illustrates a hardware set up for generation and acquisition of light emitted profile.
Figure 28 illustrates a scheme of Direct PWM.
Figure 29 illustrates a scheme of Buck PWM.
Figure 30 illustrates a structure of LEDs string.
Figure 31 is an illustrative graph of emitted light.
Figure 32 are illustrative graphs that show that the characteristic AFM-acquired signal (also called "signature") of cardiomyocytes is reproduced fairly well in an appliance according to the present disclosure.
Figure 33 is an illustrative representation of a wearable system according to the present disclosure.
Figure 34 is an illustrative structure of LEDs PCBA.
Figure35 illustrates PCBAs connection for each zone.
Figure 36 illustrates a distribution of zones with LEDs.
Figure 37 illustrates the wiring of zones and HUB towards control system.
Figure 38 depicts an illustrative layout of a control system of an appliance for stimulating a living human or animal cell either in vivo or in vitro extracted from a human or animal body.
Figures 39 to 41 show illustrative embodiments of appliances according to this disclosure, wearable by a user for stimulating a living human cell in vivo.
Figure 42 illustrates a sample embodiment of an appliance of the present disclosure realized as a wearable calf sleeve.
Figure 43 illustrates a possible arrangement of near Infra Red LEDs (NIR-LED) over the flexible printed circuit (FPC) of the calf sleeve of figure 42.
Figure 44 illustrates a sample honeycomb pattern for the calf sleeve of figure 42.
Figure 45 depicts sample lens that may be installed onto the NIR-LEDs of the calf sleeve of figure 42.

### DESCRIPTION OF ILLUSTRATIVE EMBODIMENTS

The Rationale for releasing signatures obtained from the nanomechanical characterization of cellular dynamics in the form of mechanical and/or light waves to afford targeted differentiating responses (i.e., but not limited to, cardiogenic, vasculogenic commitment), as well as resistance to hypoxia/oxidative stress, and reversal of cell aging, relies on the observation that nanomechanical, electric and electromagnetic (including light) patterns coexist and merge within the morphofunctional dynamics of cyto/nucleoskeletal elements, and signaling molecules, playing a major role in orchestrating the morphogenetic processes involved in both cell/tissue specification during embryo development, and tissue regeneration in the adulthood [47].

Consonant with this rationale, the delivery of the acquired signatures to targeted cells in vitro and tissues in vivo (wearables) has been designed through the use of Mechanical, and LED equipped Actuators (MELE Actuators).

Human induced pluripotent stem cell-derived cardiomyocytes were analyzed by a Bioscope I Bruker AFM in a temperature-controlled chamber. Silicon nitride cantilevers with square pyramidal tip with a nominal spring constant of 0.06 N/m (each tip was previously calibrated with the thermal noise method) were exploited to detect the mechanical oscillation signal from pulsating cells. Different methods were used to record the signal: constant force method (the cantilever is indenting the cardiomyocytes at constant force - in the order of 1 nN - while the feedback system delivers electric potential signals to the z-piezo scanner to keep a constant applied force, which means a constant cantilever deflection); "quasi"-constant height method (the feedback control of the Z-piezo scanner is kept at very low level not to follow high frequency signal but just very low frequency signals - lower than 0.01 Hz - and the changing signal from the photodetector is recorded). Care is taken to position the AFM cantilever in a region where a single beating focus is present in order to avoid the overlapping of two or more signals coming from different foci. In the case of the "quasi"-constant height signal acquisition, both the vertical deflection and the lateral torsion of the cantilever are acquired. In this case, the lateral signal is directly related to the traveling mechanical signal along the cardiomyocyte surface, whereas the vertical signal is related to height increase of the cell membrane due to the internal volume conservation of the cell. All the signals have been acquired exploiting the Signal Access Module of the Nanoscope IIIa controller interfaced with a digital oscilloscope. Several cycles of the cell beating signal have been acquired in each analysis session. The voltage signals from the cantilever photodetector (or the z-piezo voltage signal) are then analyzed by Fourier Transform analysis as detailed below.

### Method and Device for Deciphering cardiovascular signatures by AFM

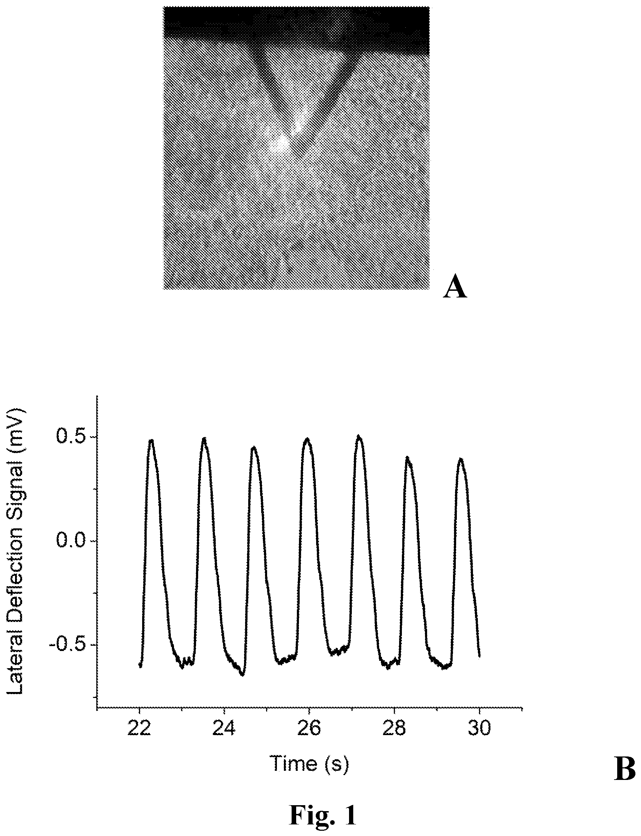

Human iPS (ATCC-CYS0105) derived from primary cardiac fibroblasts, obtained from a healthy donor and reprogrammed by the expression of OCT4, SOX2, KLF4 and MYC gene sequences using Sendai viral transduction, were cultured in defined media PSC Cardiomyocyte Differentiation kit (A2921201 - Gibco) to promote their cardiogenic differentiation. Cell persistence in culture ranged from 7 to 21 days, based upon each specific differentiation protocol. At defined time intervals, adjusted on the basis of each differentiation protocol (StemPro Differentiation Kit Gibco - A1007001 - A1007201 - A1007101), cells were probed with AFM and data were recorded (Figure 1 A, B). In particular, the electric potential difference patterns derived from the deflection and torsion of the AFM cantilever (Figure 1 B) were used to construct an AFM-derived signature, as described above (Acquiring cell signatures from the deflection of the AFM cantilever).

Figure 1 A shows an image of the AFM cantilever probing hiPS-derived cardiac myocytes at onset [approximately day 12 following the induction with cardiogenic medium (PSC Cardiomyocyte Differentiation kit)] of their acquirement of a spontaneous beating activity, indicating the attainment of a terminal cardiac differentiation. In particular, Figure 1 A shows a snapshot of a 2-min movie of the beating activity: please note the top view of the triangular shape of the AFM cantilever and the red spot of the laser beam projected onto the cantilever. The profiles of the AFM cantilever lateral torsion obtained during the cardiac differentiation were then recorded, as shown in Figure 1 B. Cantilever deflection and torsional patterns were used to construct a specific AFM-derived signature in the form of voltage signal. Such signature was then conveyed to ad hoc designed mechanical or light-emitting actuators as detailed below.

### Methods and Devices for Delivering Cardiovascular Signatures in vitro with Mechanical Actuators

A Uniaxial Stretcher, as well as an Isotropic Stretcher were developed in order to convey the AFM-derived signal to in vitro cells inside a typical cell-incubator or inside an on-top stage incubator.

CAD schemes were produced and then the different components were produced by a milling machine.

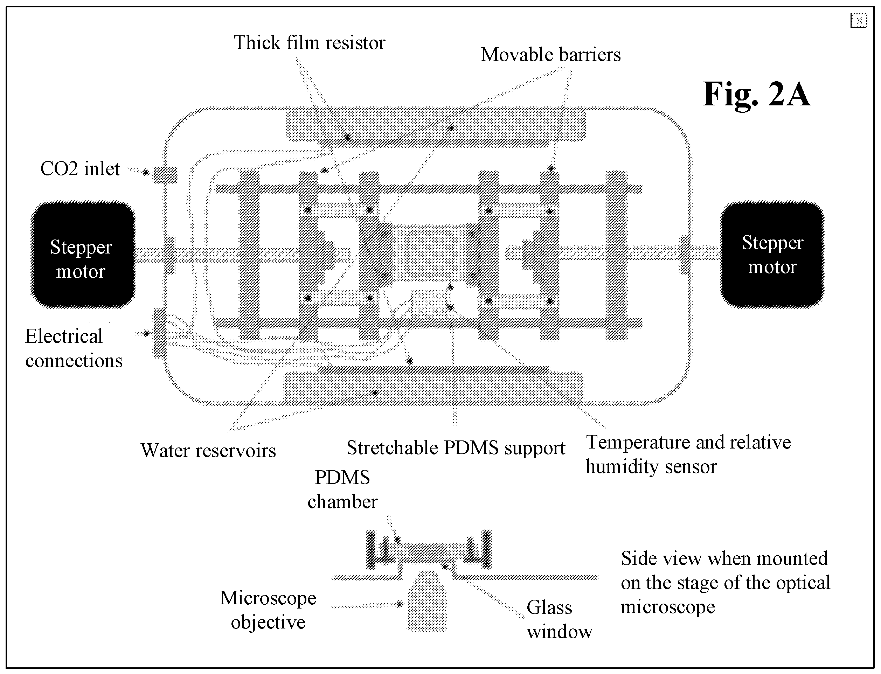

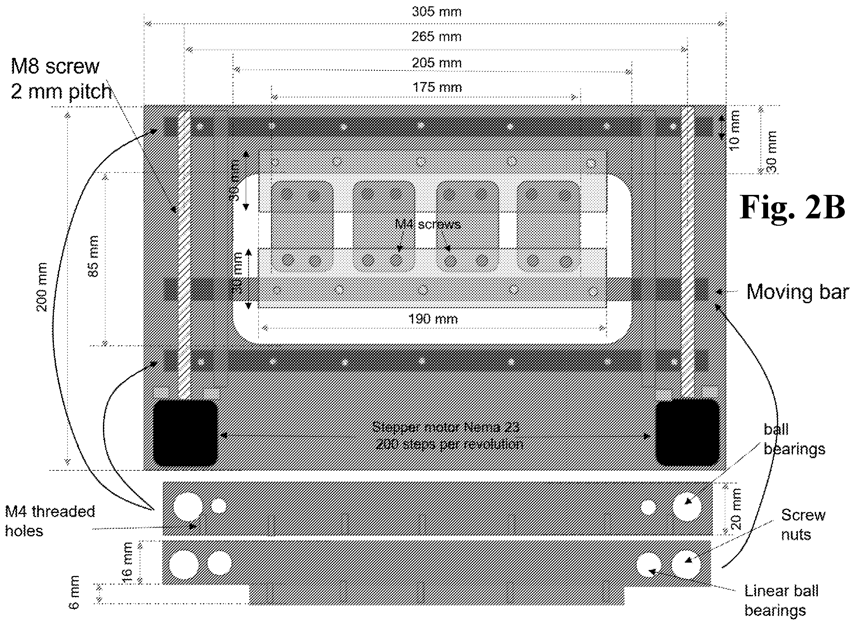

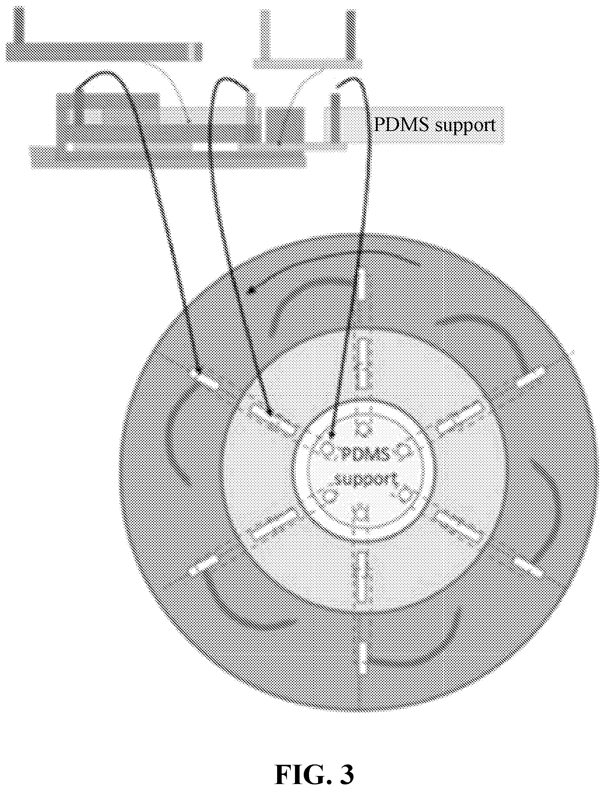

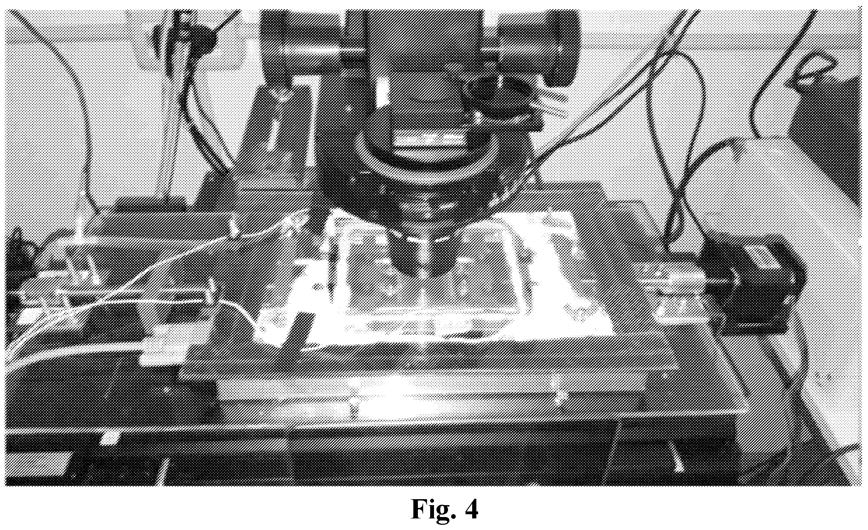

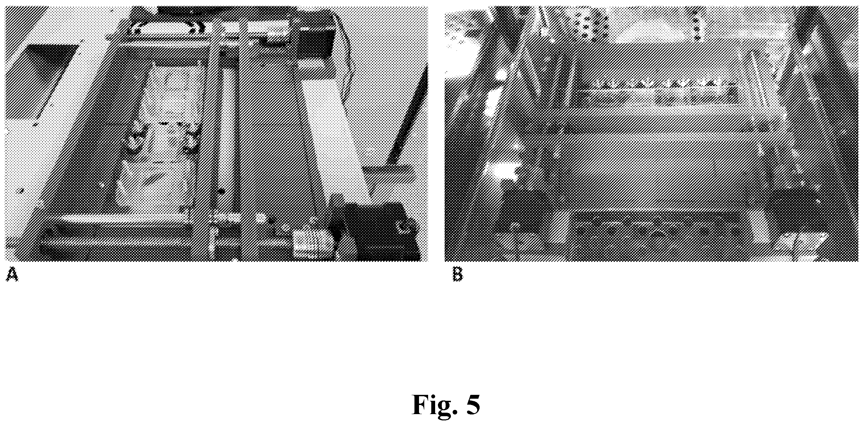

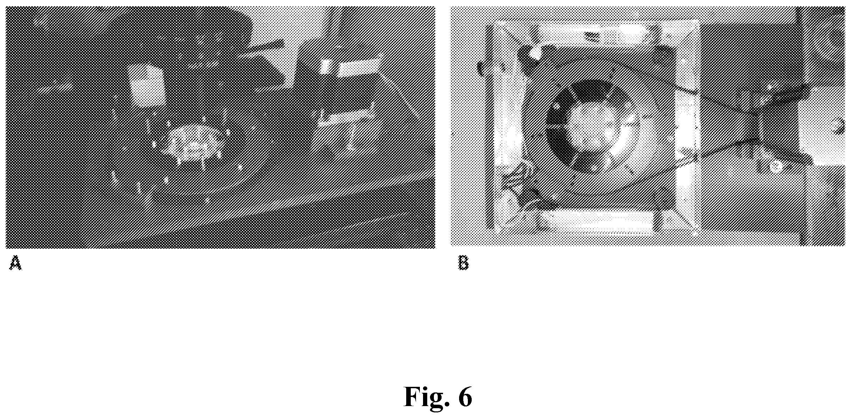

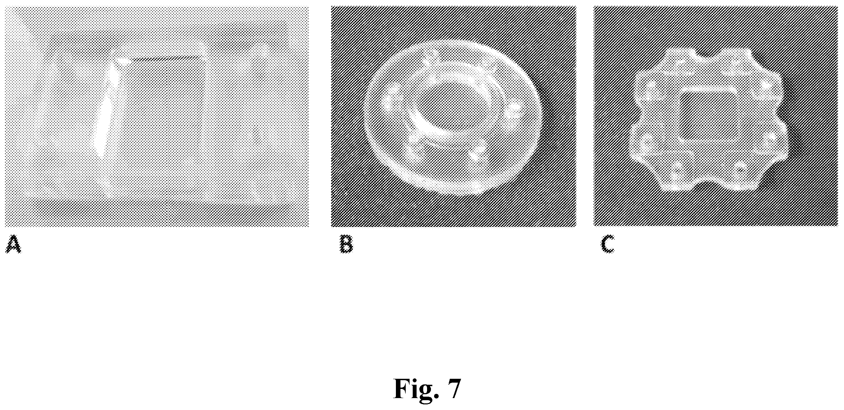

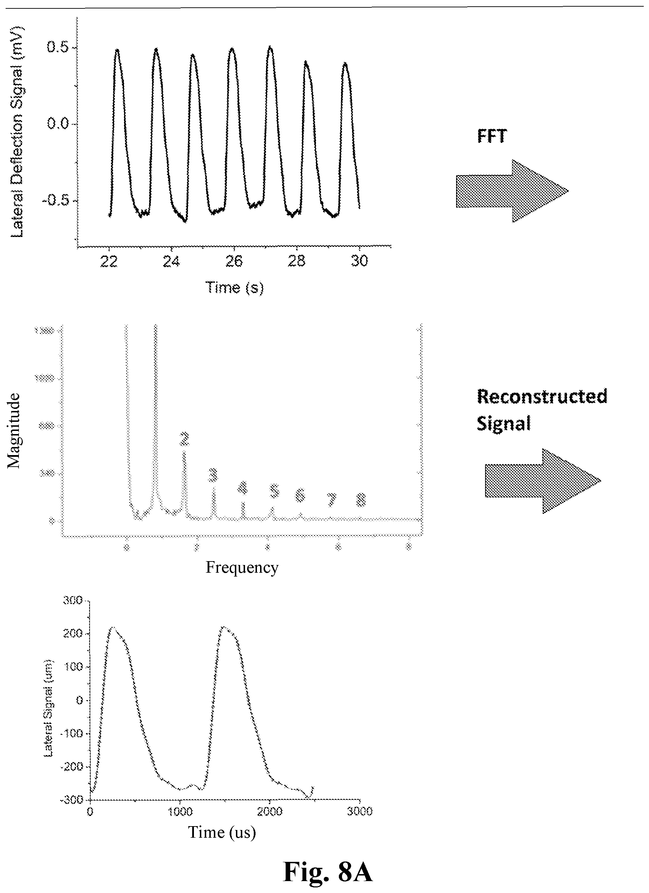

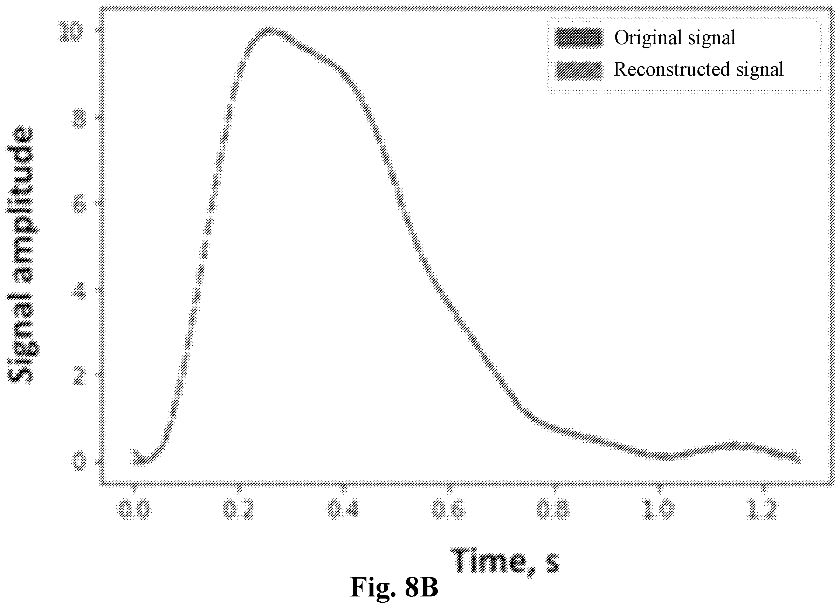

Figure 2 A, B shows the schemes of the development of the Uniaxial Stretcher, while the scheme for the Isotropic Stretcher development is shown in Figure 3. Figure 4 shows the picture of the Uniaxial Stretcher used to convey the mechanical strain signal to cells in vitro, inside an on-top stage incubator connected to an inverted optical microscope (Olympus IX 70, Tokyo, Japan). The internal conditions of the incubator were: temperature at 37°C, 90-95% humidity and 5.3% CO₂. Figure 5 (A,B) shows the pictures of the Uniaxial Stretcher inside a standard CO₂ cell culture incubator (37°C with 5% CO₂ in a humidified atmosphere). Figure 6 shows pictures of the Isotropic Stretcher on the stage of an inverted optical microscope (A), and details of the inside driving dynamics (B). Supports for the cyclic stretching protocols were fabricated using the PDMS (Polydimethylsiloxane - Sylgard 184) elastomer with a 20:1 polymer/crosslinker mass ratio. We developed different designs for the different stretching devices. Figure 7 A shows the PDMS support for the uniaxial stretching device, whereas Figure 7 B, and C shows the supports for the Isotropic Stretcher. The specific geometry of the uniaxial stretcher support was optimized to increase the uniaxiality of the strain map. In fact, the deformation of the support, even if directly applied in a unique direction, due to the Poisson effect (conservation of the volume) implies a corresponding deformation in the perpendicular direction. The strain map up to a 10% deformation in the direction imposed by the stepper motor was simulated by finite element simulation and calibrated experimentally. The 20:1 mixture of Sylgard 184 produces an elastomer with a Young modulus in the range of a few MPa. To reproduce a surface with a Young modulus comparable to the cardiac tissue (healthy tissue ≈ 5-8 kPa) we deposited on the bottom of the PDMS Sylgard 184 support a thin layer of PDMS Sylgard 527 at 1:1 mass ratio of the two components. This layer assures the right Young modulus for a healthy tissue as determined using a technique based on the micropipette aspiration of the polymer. The delivered signal was derived from the lateral cantilever torsion. This signal reproduces the mechanical communication process from cell-to-cell and could represent a synchronization stimulus among cells. The mechanical actuators (used in both Uniaxial/Isotropic Stretcher) are based on Nema 17/23 stepper motors (200 steps per revolution) driven by a DM542 driver. The motors are controlled by an Arduino Uno processor interfaced with LabView. To obtain the signal to be reproduced by the Arduino microprocessor a Fast Fourier Analysis of the signal was performed on at least 20 repetitions of the oscillation pattern as shown in Figure 8. The region of the spectrum up to the 8th order was exploited to reconstruct the specific signal to be delivered to the stretcher.

Two strategies can be implemented: the signal can be low-pass filtered including the original signal below a threshold of 7 Hz (in order to include the first 8 peaks of the spectrum); the first 8 peaks were selected and, taking into consideration their frequency, amplitude and phase, the signal was reconstructed by an inverse Fourier Transform process. Figure 8 reports the steps in the case of the first strategy. In this specific case the signal to be delivered to the stretcher was based on the repetition of two cycles of the beating process (the overall signal must be connected so as to make the initial value coincide with the final one in order to avoid a drift of the stretching deformation).

In the second strategy, whose reconstructed signal is reported in Figure 8, a single cycle was used for delivering the stretching protocol. In the second strategy, by altering the amplitude of the individual components, a scrambled signal can be easily reconstructed and conveyed by the stretcher to cells.

### Calibration of the effective strain signal on the PDMS support

### The signal conveyed

### Implementation of the stretching waveform in the driving circuit

The stretching deformation is applied to the PDMS substrate via a stepper motor by transforming a rotation movement into a linear translational movement. Every single step of the motor corresponds to 10 µm translation for a 200 steps/revolution motor and a screw with a pitch of 4 µm. The amplitude of the periodic signal to be conveyed to the substrate can be converted from the metric scale to the corresponding number of single steps of the motor and the time-interval between two consecutive steps has to be evaluated. Since the displacements provided by the stepper-motor are discretized, the periodic function is discretized as well. Starting from an arbitrary function y=f(t) with f(t) = f(t+T), where T is the period, it is possible to invert the function in order to obtain t: $t = {f}^{- 1} \left(y\right)$

Since the periodic function has been discretized, the sequence of stepper motor rotations steps can be defined, y₀=*f*(t₀), y₁=*f*(t₁), y₂=*f*(t₂) and so on, where the subscript specifies the sequential number of the step out of N steps in which the period has been divided. The boundary condition to assure periodicity is the following: ${y}_{0} = f \left({t}_{0}\right) = f \left(0\right) = {y}_{N} = f \left({t}_{N}\right) = f \left(T\right)$

Exploiting the inversion of the periodic function, we can estimate the different time intervals: $\begin{matrix}{t}_{0 , 1} = {t}_{1} - {t}_{0} = {f}^{- 1} \left({y}_{1}\right) - {f}^{- 1} \left({y}_{0}\right) , \\ {t}_{1 , 2} = {t}_{2} - {t}_{1} = {f}^{- 1} \left({y}_{2}\right) - {f}^{- 1} \left({y}_{1}\right) , … , {t}_{N - 1 , N} = {t}_{N} - {t}_{N - 1} = {f}^{- 1} \left({y}_{N}\right) - {f}^{- 1} \left({y}_{N - 1}\right)\end{matrix}$

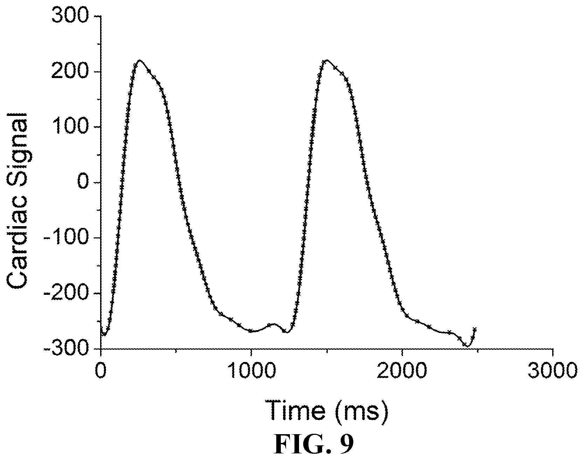

For a minimum time-interval between two consecutive steps of 4 ms, the time intervals t_{1,2}, ... t_{N-1,N} are approximated in order to be not less than this value (this condition can provide a limit to the maximum obtainable deformation rate) and they are approximated to milliseconds. Figure 9 shows the discretization we considered for the cardiac signal. The discretization allows to establish the time delay between consecutive motor steps.

### Light-Emitting Actuator

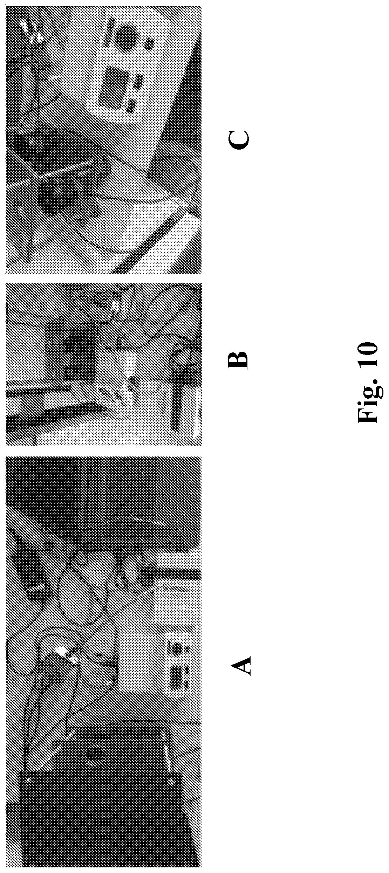

Figure 10 shows pictures of the Array of Near Infrared (NIR) emitting LEDs. The reconstructed signal collected with the AFM is transformed into a sequence of voltage pulses reproducing the final waveform. In particular, the final waveform is obtained by summing up the first eight harmonic waves obtained from Fast Fourier Transform (FFT) of the initial AFM signal. This voltage signal is required to drive the three NIR collimated LEDs (810 nm, Thorlabs M810L3-C5). In fact, the lasers are connected to a hub (Thorlabs DC4100 hub) and a LED driver (Thorlabs DC4104) which drive the lasers by an external voltage signal. The external signal is in fact our final waveform and is generated using a National Instruments multifunction-port device (NI USB-6211), in particular its analog output ports are exploited. The NI USB-6211 is connected and interfaced to PC by an usb connection using a custom made LabView software. The three NIR collimated LEDs (810 nm, Thorlabs M810L3-C5) are then positioned in the incubators, where three 35 mm Petri culture dishes are positioned at a predefined distance where the radiating power of light is maintained constant during the different experiments, varying the time of treatment and the waveform used. According to a sample embodiment, the distance between the sample and the collimator is approximately 4 cm and the emitting power density measured at this distance is about 0.7-0.8 mW/cm^2 .

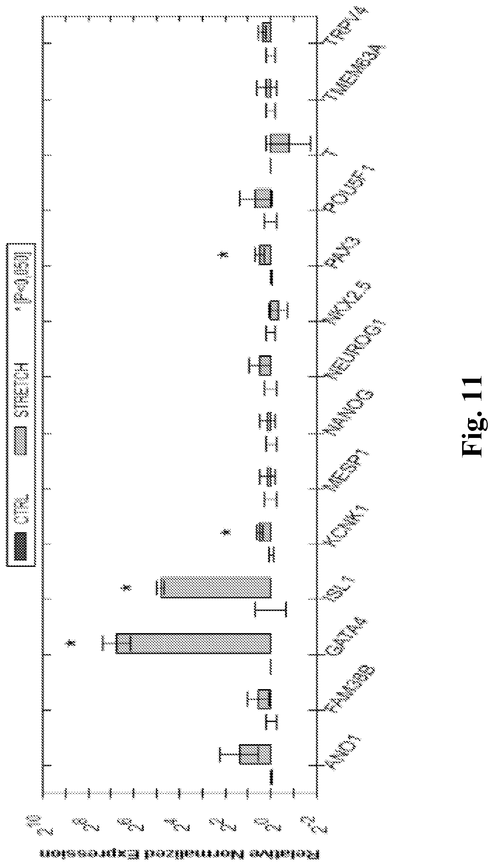

According to another sample embodiment, the maximum emitting power density is 50mW/cm^2, while the mean emitting power density is 18mW/cm^2. The signature acquired by AFM during iPS cardiogenesis was delivered in vitro by the Isotropic Stretcher described herein to hiPS cultured in basal medium (Essential 8 - Gibco) for 4 hours a day with an hour break after 2 hours, for 4 days. For this experiment, the PDMS surface is poured in order to reach a stiffness of 5 kPa, to achieve a rigidity similar to the cardiogenic tissue. At the end of cardio-stretching, hiPS were cultured in a cardiogenic medium for another 5 days without physical stimulation. Control group is maintained in the same culture conditions as the treated cells but without mechanical stimulation of any kind. This strategy resulted in a dramatic increase in the expression of cardiogenic genes GATA4 and Islet 1 (ISL1), encoding for cardiogenic transcription factors essential for cardiac development and cardiogenesis in both embryonic and iPS cells [48-51] (Figure 11). ANO1, a stemness-related gene [52], was also slightly overexpressed (Figure 11). Interestingly, these two genes were the only ones being remarkably overexpressed over a wide-ranging panel of investigated genes encoding for cardiac/vascular-related commitment (MESP1, Nkx2.5) [50], non-cardiogenic pathways, including PAX3 (skeletal myogenesis) [53], and Neurogenin1 (NEUROG1) (neurogenesis) [54], stem cell pluripotency (POU5F1, NANOG, T, TMEM63A) [55], and a variety of ion channels (FAM38B, KCNK1, TRPV4) (Figure 11).

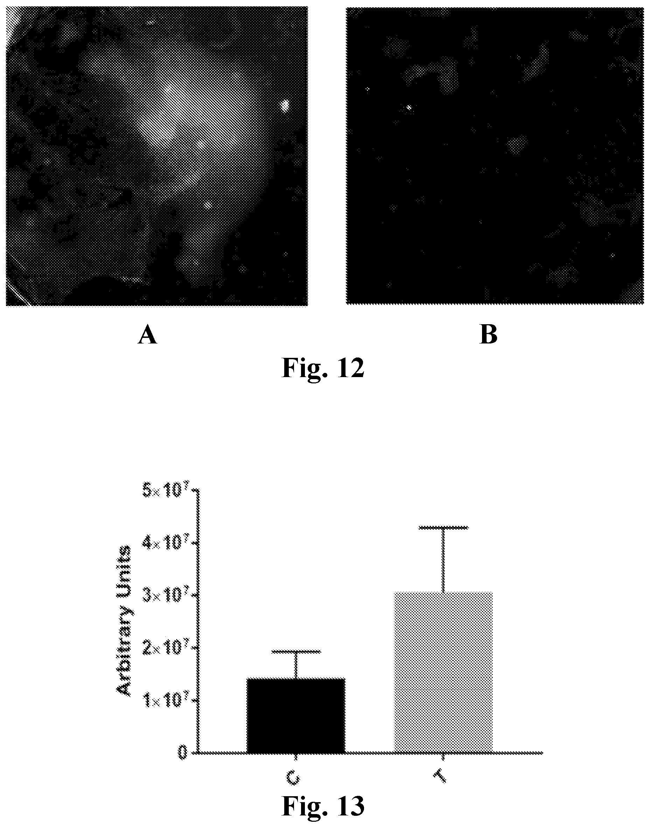

Exposure of hiPS to the AFM-acquired cardiogenic signature, as described above, elicited an early, consistent down-regulation in the expression of both GATA6 and TRA-1-60 (Figure 12 B), as compared to unexposed controls (Figure 12 A). This down-regulatory response is consistent with the observation that these two genes, and related products, are essential in creating the prerequisite for early cardiac commitment, being down-regulated throughout the acquirement of the cardiac phenotype [56-59]. The increase in GATA4 and ISL1 observed in hiPS that had been exposed to the cardiogenic signature was associated with an increase in the protein expression of α-sarcomeric actinin, a cardiac specific marker (Figure 13).

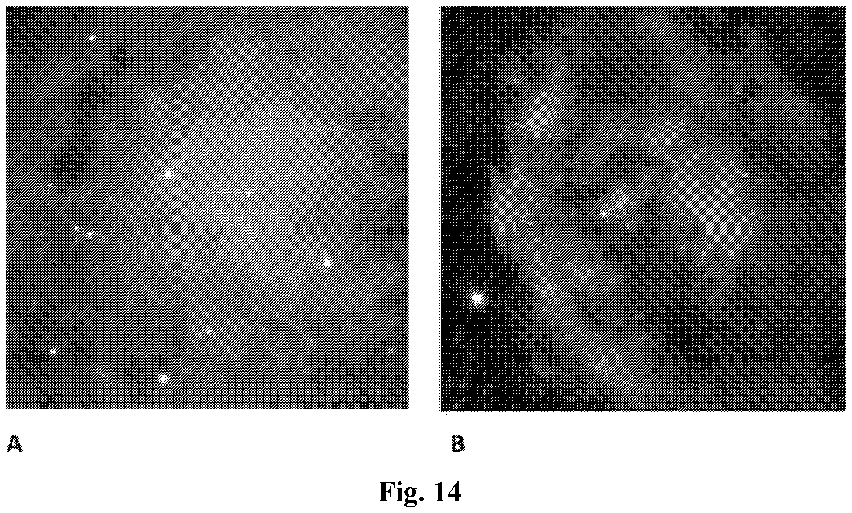

The cardiogenic action of the AFM-acquired signature was further confirmed by the finding that exposed hiPS exhibited a consistent increase in the expression of cardiac troponin T (TnT), another major cardiac-specific marker (Figure 14 B), when compared to unexposed cells (Figure 14 A).

### Inducing vasculogenesis by the Delivery of AFM-acquired Cardiogenic Signature through a Light-Emitting Actuator

The signature acquired by AFM during iPS cardiogenesis was delivered in vitro to human umbilical vein endothelial cells (HUVECs) for 3 hours until the fourth day. After the light pre-treatment, cells were seeded at a density of 30,000 cells/cm² in a 96-well plate coated with semisolid medium (BME, Trevigen). Cells were maintained in basal medium (DMEM, 2 mM Ultraglutamine, 1% Penicillin-Streptomycin solution).

The plate was introduced into the incubation chamber of the microscope station to maintain CO₂, temperature, and humidity in controlled conditions, and time-lapse acquisitions were performed over 24 h with a Nikon Inverted Microscope Eclipse Ti-E. Total well areas were captured, and tube length was measured using NIS-Elements software tools.

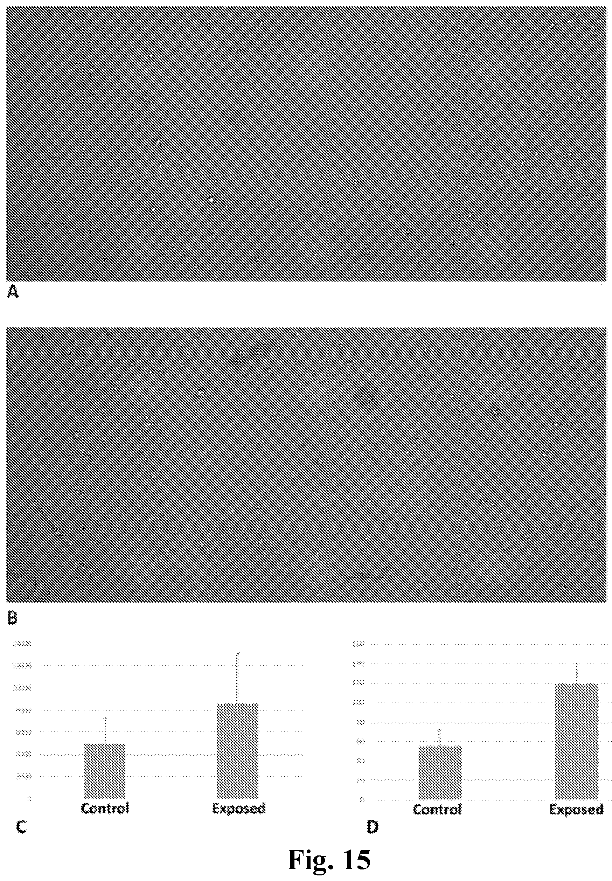

Figure 15 shows that signature-exposed HUVECs cultured in semisolid medium exhibited a remarkable increase in capillarogenesis (B), as compared to unexposed control cells (A). In particular, HUVEC exposure to AFM-derived cardiogenic signature significantly enhanced the capillary length (C), and the capillary branching (D).

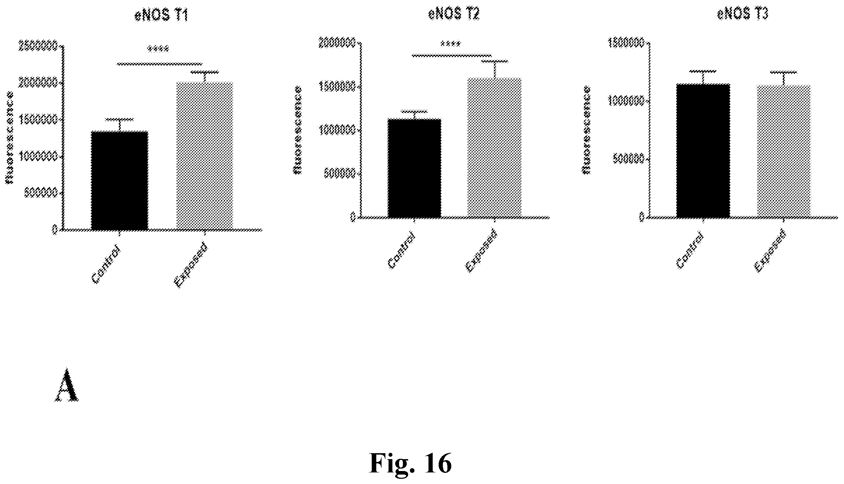

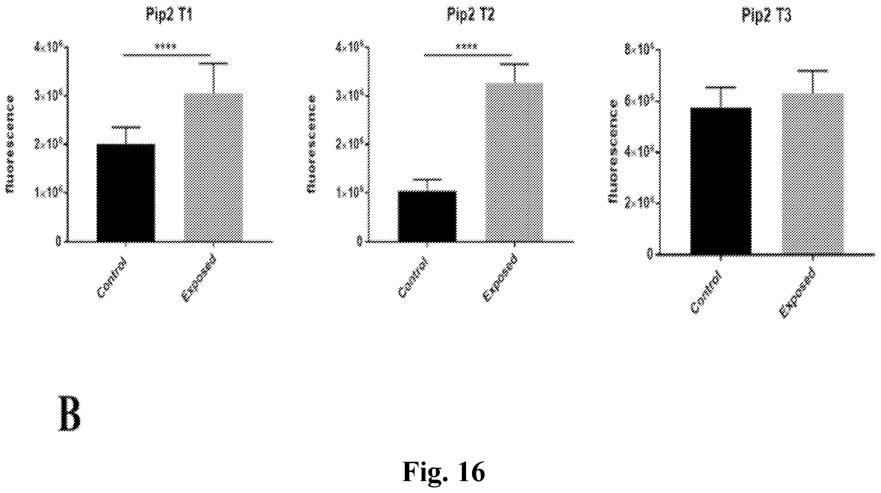

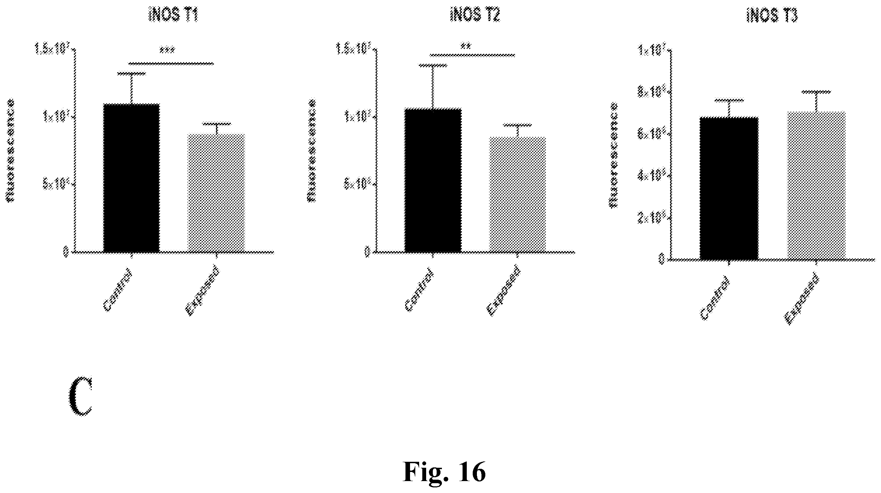

Immunofluorescence data showed that during the first 24 hours of exposure to Light-Emitting Actuator, there was increased expression of the vasculogenesis-related protein eNOS and Pip2, a specific regulator of ion channels directly involved in vasculogenesis, in cardiogenic signature-treated HUVECs (Figure 16, A and B, T1). This overexpression persisted for the next 24 hours (T2), and was lost after 3 days (T3). At the same time, iNOS was downregulated in treated cells (Figure 16, C). It has been shown that iNOS in HUVECs is related to cell culture stress and not to vasculogenesis, therefore its underexpression is a sign of a better metabolic condition induced by cardiogenic signature in the treated cells compared to control cells.

### Enhancing hMSC capabilities by Delivery of AFM-acquired Cardiogenic Signature through a Light-Emitting Actuator

Difficult tissue healing, resulting from vascular diseases or trauma, is a major threat for the quality of life and it is becoming an emerging problem in the geriatric population.

The signature acquired by AFM during iPS cardiogenesis was delivered in vitro by the aid of a Light-Emitting Actuator to dermal human mesenchymal stem cells (hMSCs) for 3 hours a day until the fourth day. After the light pre-treatment, when hMSCs reached confluence, they were scratched by a 100-µL sterile pipette tip. Images of the whole wound were taken after the scratch and 22h post-treatment with a Nikon Inverted Microscope Eclipse Ti-E. Scratch areas were measured at 0 and 22h, and scratch closure percentages were calculated using NIS-Elements software tools.

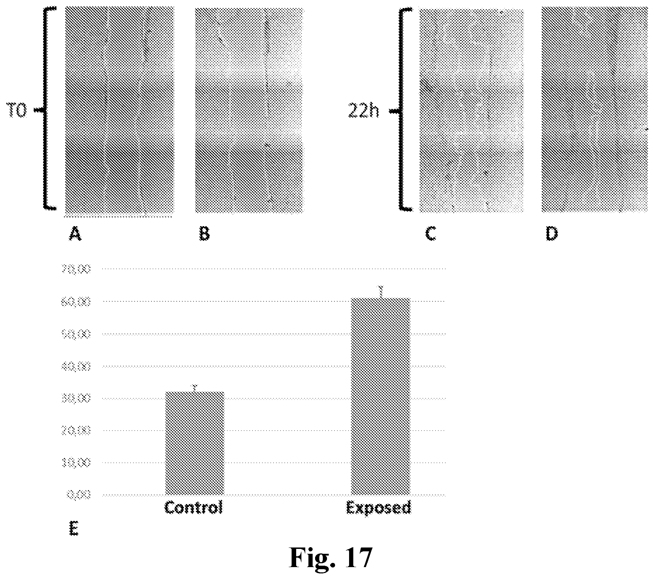

Figure 17 shows representative images of scratch closure after 22h in control unexposed hMSCs (A) or in hMSCs that had been pretreated for 3h a day until the fourth day with the AFM-acquired cardiogenic signature. Images of the whole wound were taken after the scratch and 22h post-treatment with a Nikon Inverted Microscope Eclipse Ti-E.

Scratch areas were measured at 0 and 22h, and scratch closure percentages were calculated using NIS-Elements software tools. Light exposed hMSCs exhibited a remarkable increase in the percentage of scratch closure.

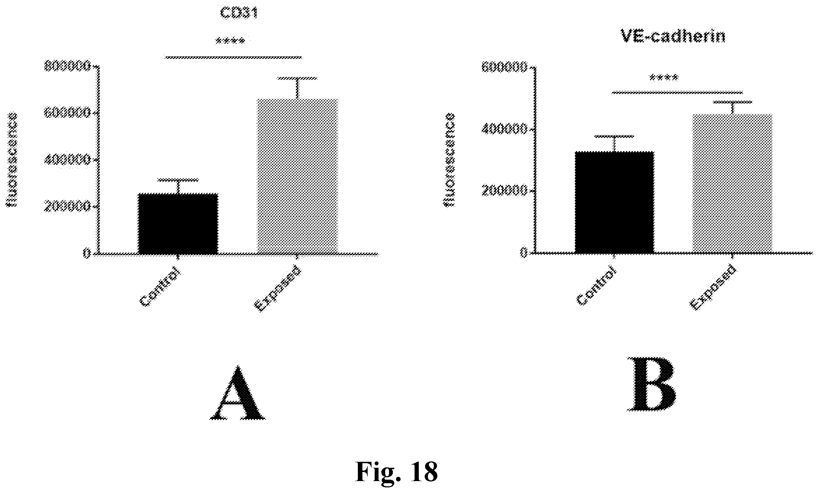

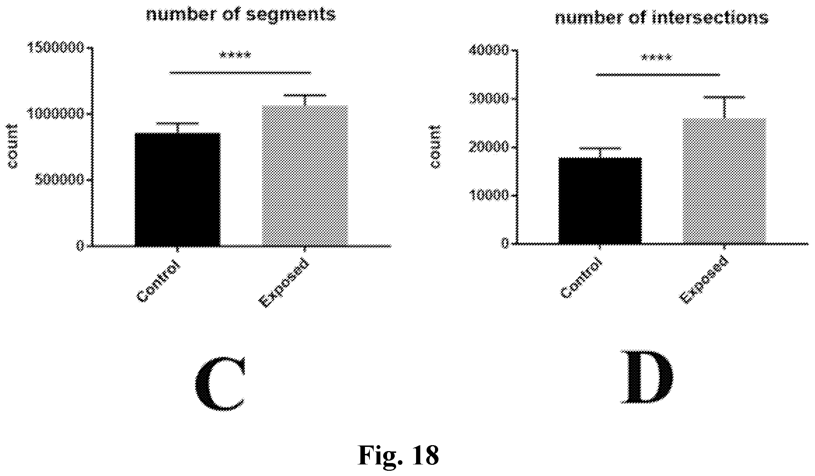

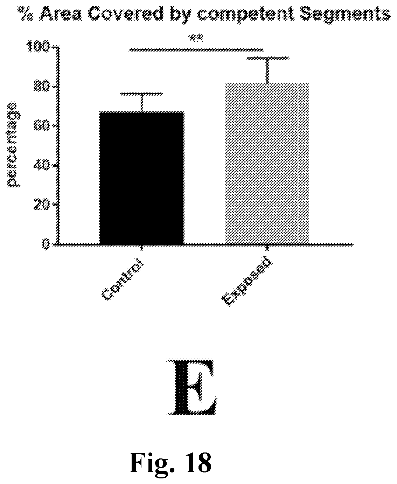

In an additional set of experiments, HUVECs and hMSCs were treated with the aid of a Light-Emitting Actuator for 5 days, then detached and co-cultured for additional 3 days, with the light treatment continuing throughout. Cells were seeded at a concentration of 40,000 cells/cm² for hMSCs and 45,000 cells/cm² for HUVECs. A medium consisting of 50% DMEM LG with 5% FBS and 50% EBM-2 with 1% Penicillin-Streptomycin solution was used in the co-culture plates. At the end of the treatment, the cells were fixed and angiogenesis was evaluated by staining with VE-cadherin and CD31 antibodies. Figure 18 shows that the cardiogenic signature was able to induce CD31 and VE-cadherin protein expression, leading to a more sustained angiogenesis, as measured by the percentage of area covered (A), number of segments (B), and junctions between vessels (C).

In a separate set of experiments, we assessed the effect elicited by hMSC exposure to the AFM-acquired cardiogenic signature on the in vitro expression of profibrotic players.

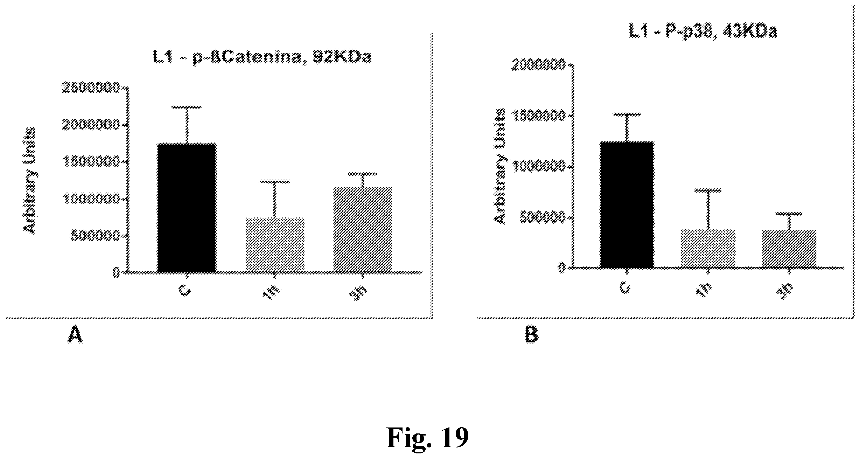

It is now emerging that β-catenin signaling is crucial for the onset of tissue fibrosis [60], and myocardial fibrosis developing in the course of myocarditis [61,62]. Moreover, Angiotensin II (Ang II)-Angiotensin Receptor Type 1 (ATR1) signaling, and the accumulation of β-catenin have been found to be critical for development of post-inflammatory fibrotic remodeling of the heart and dilated cardiomyopathy, two events resulting from the activation of profibrotic Wnt/β-catenin pathway and the accumulation of β-catenin in inflammatory regions of the heart [63]. Activated (phospho) p38 MAP Kinase also plays a major role in cardiac fibrosis and remodeling, as it is indicated by the finding that inhibition of p38 signaling reduced transforming growth factor-β signaling-related profibrotic and hypertrophic gene expression, and blocked exaggerated cardiac remodeling [64-67]. Figure 19 shows that hMSC exposure to the AFM-acquired cardiogenic signature by the aid of a Light-Emitting Actuator resulted in significant down-regulation in the gene expression of both β-catenin (Figure 19 A) and phospho-p38 MAP Kinase (Figure 19 B), as compared to control unexposed cells.

### Reprogramming human cardiac fibroblasts and extracellular matrix composition

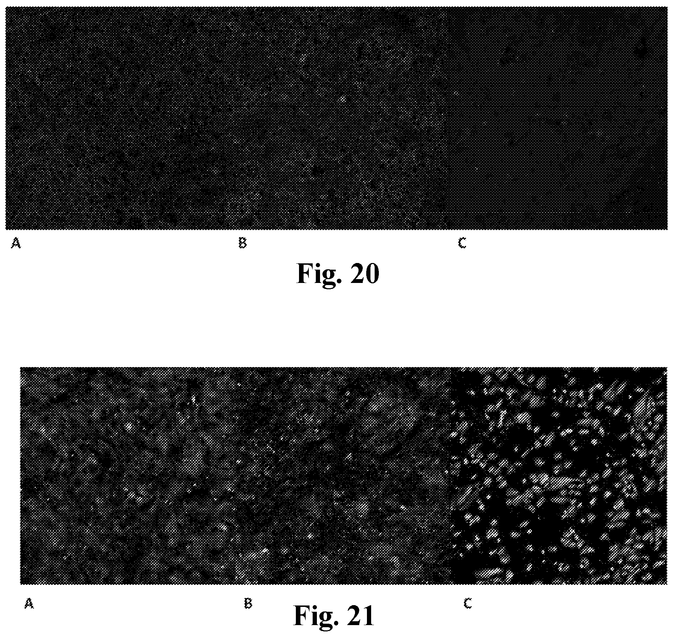

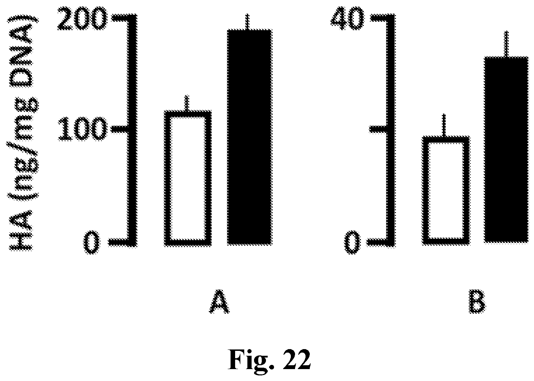

Myocardial infarction (MI) is among the leading causes of death worldwide. Following MI, necrotic cardiomyocytes are replaced by a stiff collagen-rich scar which results from a temporal maturation of post-infarct scar, with local resident fibroblasts undergoing multilineage reprogramming and transition into myofibroblasts and matrifibrocytes [68]. These complex and still partially unexplored phenomena lead to the establishment of large acellular zones exhibiting cartilage- and tendon-like features, at the level of both cell transcription patterns and extracellular matrix (ECM) composition [68]. While such adaptogenic responses result in decreasing the risk of immediate post-infarct cardiac rupture, the progressive extension of myocardial scar and its inherent stiffness are crucial determinants of the onset of diastolic dysfunction and progression towards heart failure [68,69]. Reducing collagen deposition and crosslinking, as well as myofibroblast activation has gained attention as a promising strategy for cardiac repair and regeneration, resulting from a reduction in the size of post-infarct scar and normalization of contractile dynamics [69,70], thus reversing cardiac remodeling and failure. Nevertheless, these strategies have been exploited in rodent animal models of MI through the intramyocardial delivery of targeted antibodies or molecules [69,70] that may reduce the scar stiffness. These are cumbersome and invasive approaches that are not readily envisionable in humans, in consideration of the risky context of an acute myocardial accident. Here, we show that in vitro exposure of human cardiac fibroblasts (hCFs) to the AFM-acquired cardiogenic signature by the aid of either the Isotropic Stretcher, or the Light-Emitting Actuator described herein, was able to:
- Enhance fibronectin expression (Figure 20), while producing a concomitant decrease in osteopontin expression (Figure 21).
- Elicit a switch in the expression of type I towards type III collagen.
- Increase the cellular and secreted amounts of hyaluronic acid (HA) (Figure 22).

Briefly, cells were seeded onto a PDMS mold coated with fibronectin and left inside an incubator.

When the culture reached full confluence, the molds were positioned onto the stretcher inside an incubator for 24 hours, with or without mechanical treatment. Independent from the signature used, the maximum stretch in each direction was 10%. Treatment with the cardiogenic signature resulted in remarkably higher fibronectin deposition and stronger cellularization onto the surface of a PDMS substrate (Figure 20 B), as compared to untreated control cells (Figure 20 A). Intriguingly, hCF exposure to a scrambled vibrational pattern derived from the original signature induced cell damage and scarce fibronectin deposition as well as cell loss (Figure 20 C). This finding not only revealed the effectiveness of the cardiogenic signature in modifying the ECM composition, but provided evidence that the cellular response was specific in nature, as shown by the detrimental outcome of the exposure to a scrambled vibrational pattern.

The exposure to the cardiogenic signature elicited a robust decline in osteopontin deposition, but increased cellularization onto a PDMS substrate (Figure 21 B), as compared to untreated control cells (Figure 21 A).

Conversely, hCF exposure to a scrambled vibrational pattern derived from the original signature induced cell damage, but consistently higher osteopontin deposition, as well as cell loss (Figure 21 C).

Superimposable results to those reported in Figures 21 and 22 were obtained following hCF exposure to the AFM-acquired cardiogenic signature delivered by a Light-Emitting Actuator (not shown).

Besides confirming the specific nature of the elicited responses, as shown in Figure 21, these findings demonstrate that cell exposure to the cardiogenic signature did not simply produce a generalized upregulation of matrix elements, but resulted into a differentially expressed pattern of players. To this end, there is now compelling evidence that the modulatory targeting of specific signals to endogenous cells, rather than efforts directed to transplant and integrate therapeutic cells, may result in better outcome in wound-healing approaches, including those aimed at regenerating infarcted hearts. In this regard, fibronectin is a multidomain, high-molecular-weight glycoprotein, with low-expression levels within the ECM of a healthy myocardium. While collagen I and III and laminins gradually increase from the fetal age to the adulthood, fibronectin has been found to decrease with development [71]. On the contrary, fibronectin is strongly upregulated in the infarcted myocardium [72]. Moreover, fibronectin has been shown (i) to promote highly efficient cardiac differentiation of human embryonic stem cells, in combination with laminin [73], (ii) to be essential for reparative cardiac progenitor cell response after MI [72], (iii) to act as the cleavage substrate (at the level of its type III domain-containing protein 5 (FNDC5)) for the generation of Irisin, a newly identified hormone and cardiokine, that has been shown to promote cardiac progenitor cell-induced myocardial repair and functional improvement in infarcted hearts [74]. There is now evidence that even adult mice cardiomyocytes can be funneled into a proliferative state, with viral vector-mediated expression of defined signaling molecules, or with the modulation of specific signaling pathways [75,76]. Within this context, the short-term induction of fibronectin and increased cellularity achieved by hCF exposure to the cardiogenic signature may provide an optimal microenvironmental topography for adult cardiomyocyte proliferation and cardiac regeneration. Such regenerative outcome may be reinforced by the favorable microenvironmental cues provided by the concomitant down-regulation in the expression of osteopontin, a glycoprotein implicated in a variety of acute and chronic inflammatory responses and poor long-term outcome in patients with MI [77]. In separate experiments, we used an ELISA assay kit to assess the amount of Human Collagen Type I or III (myobiosource.com). Noteworthy, the expression of type I collagen decreased by 35.4 ± 6.1 % (mean ± SEM, n = 4), while that of type III collagen was enhanced by 40.0 ± 9.2 % (mean ± SEM, n = 4), in hCFs exposed to the isometric stretcher-delivered cardiogenic signature, as compared to the expression in unexposed controls. Similarly, when the cardiogenic signature was delivered through a light-emitting actuator, type I collagen decreased by 42.0 ± 10.5 % (mean ± SEM, n = 4), with a concomitant increase in type III collagen of 44.3 ± 11.0 % (mean ± SEM, n = 4) in exposed hCFs, in comparison to non-treated control cells. Such a shift in the ECM composition of hCFs may have remarkable functional biomedical implications. In fact, an increase in type III collagen deposition has been shown to confer a dramatic reduction in the stiffness of the extracellular environment [78,79]. Moreover, type I collagen presumably opposes sarcomeres overstretching and determines tissue stiffness in post-infarct scarring myocardium. In contrast, type III collagen fibers have a lower tensile strength compared to type I collagen, and have been proposed to be more suitable to maintain structural integrity of collagen network, and to be associated with lower myocardial stiffness [79-81].

The significant increase in hyaluronan accumulation in the ECM of exposed hCFs is a relevant outcome, since we have previously shown that hyaluronan, delivered in the form of mixed esters with butyric and retinoic acids, afforded myocardial survival and repair without stem cell transplantation [82]. This finding is consonant with subsequent studies showing that: (i) hyaluronan hydrogels reduce post-infarct myocardial strain and infarct expansion [83], (ii) HA and its receptor Hmmr are required during heart regeneration in species like the Zebrafish with intrinsic ability to regenerate the injured heart [84], (iii) in the zebrafish heart, a large, regeneration-associated cluster of cells exists, expressing genes encoding the hyaluronic acid (HA)-organizing factors hapln1a and hapln1b [85]. These cells envelop proliferative cardiomyocytes during key cardiogenic events, during both heart morphogenesis and rescue, when they are required for normal HA organization in cardiac injury sites, enabling normal cardiomyocyte proliferation and muscle regeneration [85], (iiii) hyaluronan oligosaccharides (o-HA) could reduce infarct size and apoptosis in the MI region, also promoting myocardial angiogenesis and myocardial function reconstruction in MI mouse model [86], (iiiii) o-HA also improved polarization of M2 type macrophages, and removed the inflammatory response caused by neutrophils for accelerating myocardial function reconstruction in vivo [87], (iiiiii) injectable hyaluronic acid based microrods provide local micromechanical and biochemical cues to attenuate cardiac fibrosis after MI [88]. On the whole, these findings indicate that the cardiogenic signature either delivered mechanically by the Isotropic Stretcher, or in the form of electromagnetic radiation with the light-emitting actuator was able to afford a reprogramming in the ECM compositions towards a softer environment, which will be essential to oppose the progressive increase in myocardial stiffness and fibrosis occurring in post-infarct myocardial scarring. Moreover, the increase in hCF cellularity observed in the exposed cells may prove rewarding in re-installing a more suitable mechanobiology in infarcted hearts, since hCFs are now emerging as the main mechano-sensing, mechanotransducing elements in the normal heart, providing essential mechanobiology cues in mechanoelectric myocardial coupling [89,90]. To this end, the increase in number of a hCF population committed to the generation of a softer, more compliant mechano-electric environment may be the equivalent of an endogenous surgical suture, bridging the remnant post-infarct myocardium and designing a nano/micro-topography suitable for re-population of de-novo myocardium generated by proliferating cardiac myocytes, or contributed by cardiac progenitor cells whose exact nature and rescuing potential will require further investigation.

### Methods for delivering morphogenetic signatures in vivo

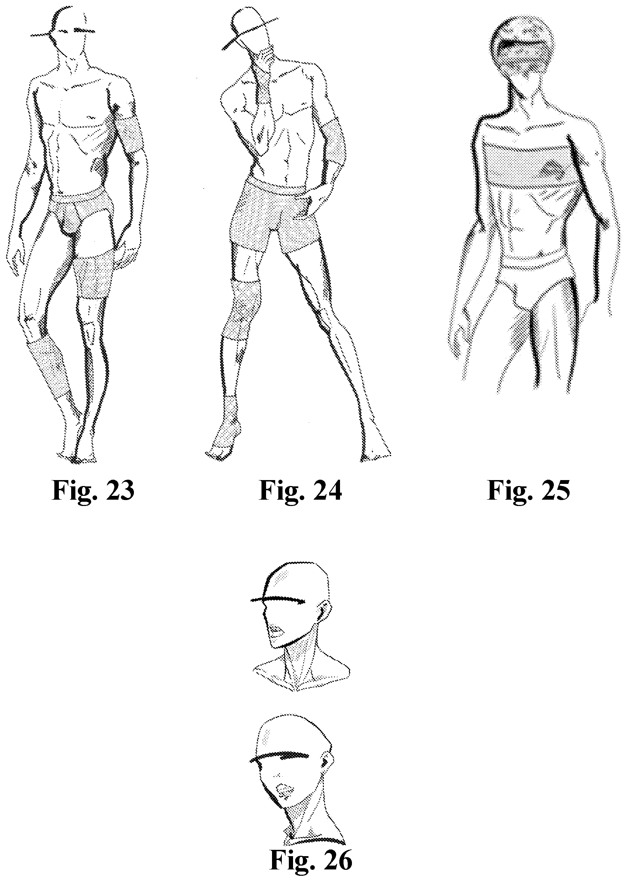

In another embodiment, we present ELVINN (Eldor Vitality Innovative Nets), a new generation of Wearables, of the type shown for illustrative purposes in Figures 23 to 26, embedding NIR LED arrays (but not limited to the NIR spectrum domain) as the light-emitting actuator, and Piezo arrays within a polymeric structure, encompassing a flexible conductive material.

Each of these actuators will deliver the AFM-acquired cardiogenic signature individually, or in a combinatorial fashion, thanks to a microprocessor unit, powered by a replaceable, rechargeable battery, under the remote control by a software program executed through smartphone/tablet compatible apps, which form part of the present invention, as described below.

The cardiac differentiating and vasculogenic responses elicited by the AFM-acquired cardiogenic signature will be exploited by ELVINN to target multiple tissues in vivo, to enhance their ability to cope with:
1. Heart Failure 2. Vascular Diseases/Erectile Disfunction/Ovarian Rejuvenation 3. Muscle Impairment/Chronic Fatigue 4. Neurodegeneration/Cognitive Impairment emerged from Cerebral Vascular Disease 5. Osteoporosis, non-Healing Fractures, Osteoarthritis, Joint Impairment, resulting from impaired bone/tissue vascularization 6. maxillary and mandibular bone resorption, and periodontal disease 7. Aging, ensuing from impaired tissue perfusion.

These treatments are conceived as regenerative well-being approaches, capable of enhancing our intrinsic capability for self-healing and coping with tissue derangement.

ELVINN will be fashioned as a family of Wearable embedding actuators designed to adapt to different parts of our body, based upon the desired regenerative well-being outcome, as it is described below.

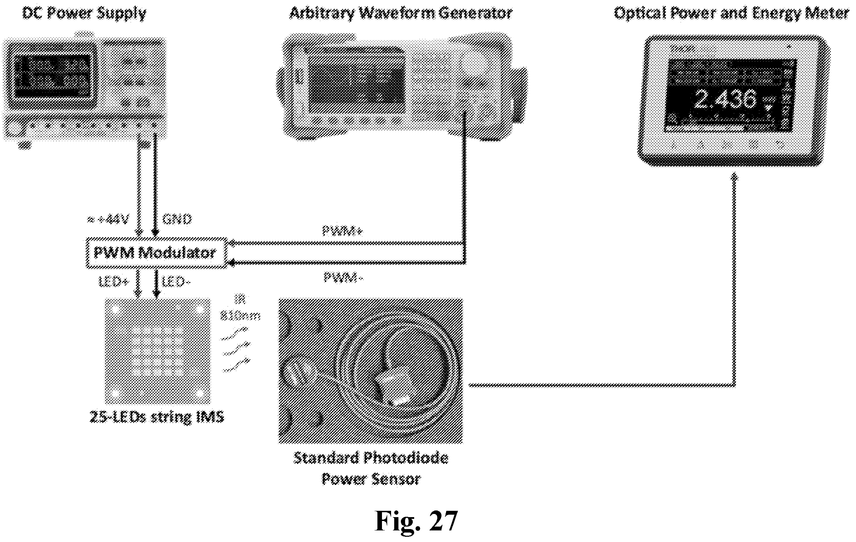

System described in Figure 27 is used in the verification phase. The collected and processed signatures are faithfully remodulated through a generator of arbitrary functions towards a matrix of LEDs.

In addition to the generation, the method used to verify that the light profile emitted by the LEDs is actually the expected one described using a photodiode and an optical power meter from Thorlabs.

This system is capable to send an IR-signal, from a custom-made 25-LEDs string IMS to a photodiode power sensor and, through an optical power and energy meter, we can see the output optical power of the modulated signal.

The PWM modulator is required in order to stimulate the 25-LEDs string IMS with a modulated signal, with the help of an arbitrary waveform generator, in which is loaded the specific signal that brings the information.

The DC Power Supply is used to feed the PWM modulator, and so, the 25-LEDs string, which requires almost 45V.

The System functionalities can be summarized as follow:
▪ PWM MODULATION: the PWM Modulation can be performed in two ways (Direct PWM or Buck PWM), both explained in a separate paragraph below. The idea is to use modulation to detect different "cell signatures" and to extract the information from those signals.
▪ IR-SIGNAL DETECTION: the 25-LEDs string IMS emits IR signals, with wavelengths of 810nm, that are detected by the photodiode. This latter converts light energy into electrical one, when incident light falls on it: actually, it generates current when it absorbs photons.
▪ WAVEFORM ANALYSIS: once we have the photodiode output signal, the optical power meter can display the output optical power. Furthermore, with all the possible options of this instrument, we can see also the graph of the modulated signal.

The PWM modulation can be performed in two ways:
1) Direct PWM. In this solution, the 25-LEDs string is controlled, and so modulated, directly by the power MOS-FETs included in the circuit, controlling directly the ON/OFF phases of the LEDs: it's important to notice that, when a LED is switched ON, it will be at maximum brightness.

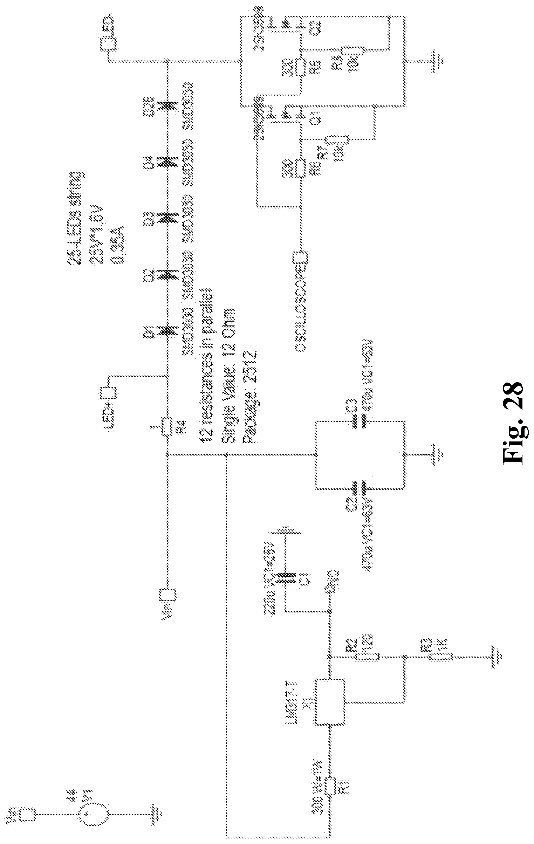

An illustrative electronic scheme is presented in Figure 28.

The LM317-T is an adjustable regulator, used to limit the current (internally has built-in current limiting), and to provide a low-dissipation power supply (in this case is not connected): in this case, a portion of the supply current will flow through the regulator and another one through the capacitors connected to ground, so the 25-LEDs string will see only a part of the total current.

The current mirror is realized with the power MOS-FETs 2SK3599: the purpose of this structure is to obtain a real current generator, with low input impedance, having at the output a better current source with higher impedance.

NOTE: In the scheme are presented only 5 SMD3030, but the total LEDs string includes 25 of them.

The main drawback of this solution is the possible difference between the time integration constant of the cell and the carrier signal frequency: this can cause a problem when the carrier signal should follow the modulating one.

2) Buck PWM. In this solution, there is a buck converter that controls, and so modulates, the 25-LEDs string: differently from the direct PWM here, when a led is switched ON, it is always ON and changes its brightness depending on the different signals in input (in our case we consider the different "cell signatures").

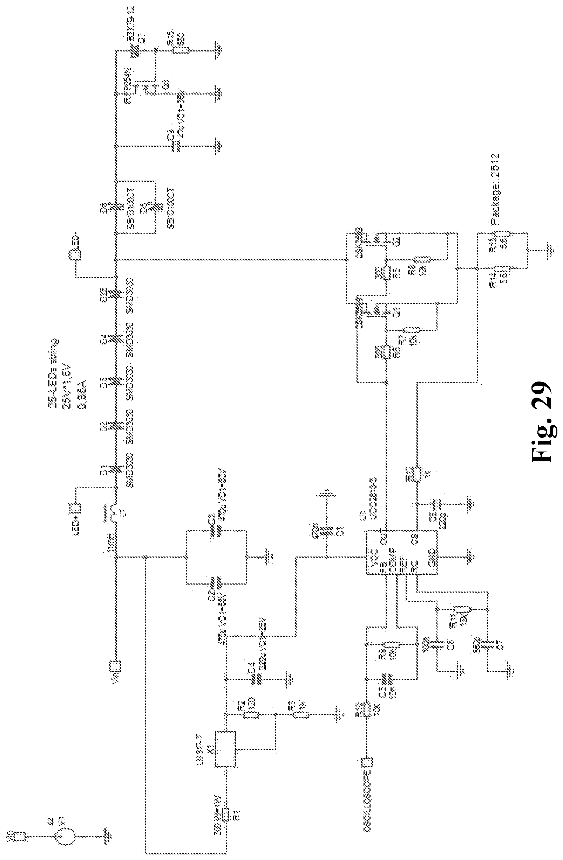

An illustrative scheme is presented in Figure 29.

The PWM operation is held by the UCC2813-3, and there is a coil of 11mH to perform buck operations.

Furthermore, there are an IRFP054 and a Zener diode of 12V, in order to clamp the voltage to maintain the inductor in continuous mode.

Apart from the previous differences, the rest of the circuit is the same of the direct PWM one.

NOTE: In the scheme are presented only 5 SMD3030, but the total LEDs string includes 25 of them.

The main drawback of this solution is to know exactly the specific activation energy interval in which the cell is stimulated and is not destroyed. The difference is that in the direct PWM solution, when a LED is switched ON, it will be at the maximum brightness, while in the Buck PWM architecture the brightness changes according to the cell signature.

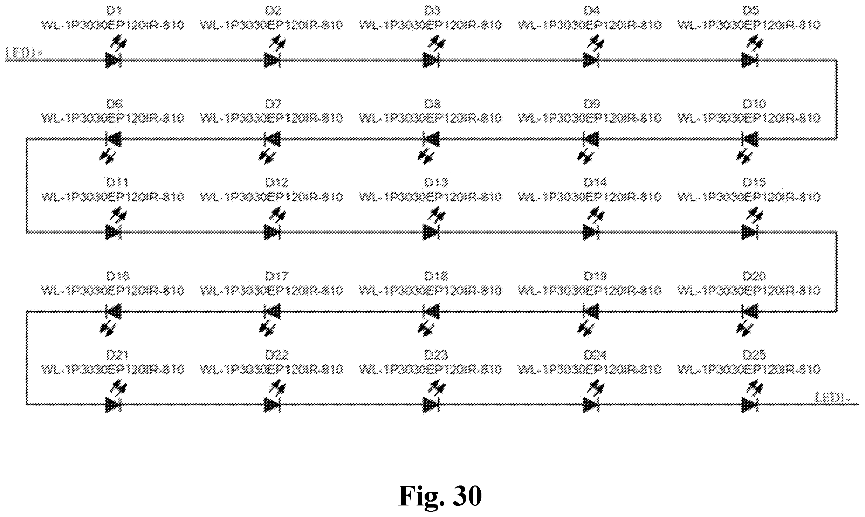

An illustrative structure of the 25-LEDs string IMS is represented in Figure 30.

The LEDs used are high power devices (1W), with wavelength of 810nm: WL-1P3030EP120IR-810.

Each of them is supplied by 1.7V (nominal) with 350mA (max), while for the operating conditions the table below is presented:

| **Characteristics** | **Unit of measurement** | **Performances** |
|---|---|---|
| Operating Temperature Range | °C | -25 ÷ 85 |
| Working temperature | °C | <50 |
| Storage Temperature Range | °C | -35 ÷ 100 |

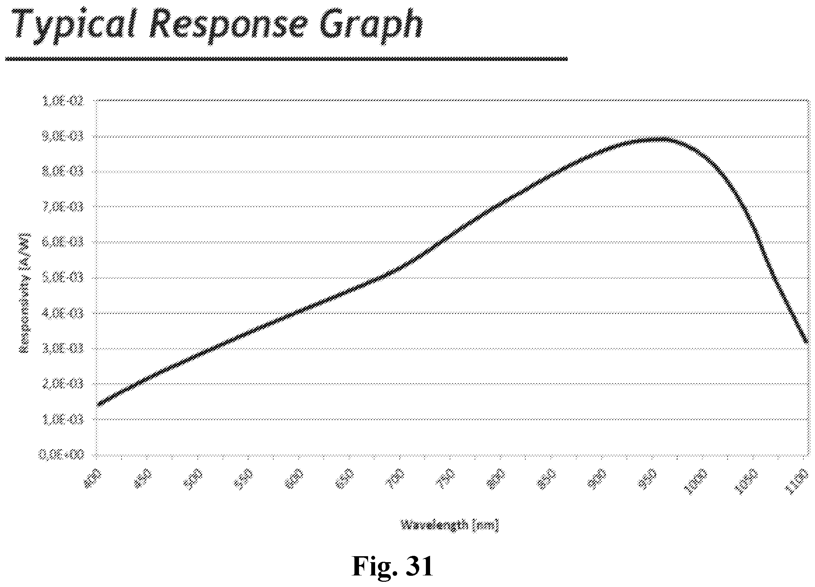

The photodiode is a Compact Photodiode Power Head with Silicon Detector S121C, used in combination with an Optical Power and Energy Meter PM400, both of them provided by Thorlabs: 1) Photodiode. First, the photodiode is set to zero before its usage, and then the λ value is set to 810nm: in this way the sensor will follow the responsivity curve in a precise working point (Figure 31). 2) Optical Power and Energy Meter. The optical power meter is used to detect the optical power of the modulated signal, in order to analyze its value in different situations. With this instrument, we can have three different display modes: - Needle Screen, with analog view of data - Numerical Display, with digital view of data - Graph display, with graphical view of data - Statistics Display, with statistic values of the samples detected - Recording of the amplified analog output voltage from the console, using an oscilloscope.

In order to feed the PWM module (direct or buck) and so, the 25-LEDs string IMS, these specifications may be followed:

| **Parameter** | **Unit of measurement** | **Value** |
|---|---|---|
| Voltage Supply | V | 44 |
| Current Supply | A | ≤1A |

This because we have 25 LED diodes with a typical forward voltage of 1.6V, with continuous forward current of 350mA (maximum rating). Furthermore, we must also consider the absorption of the PWM circuit (direct or buck).

Placing the Silicon Detector S121C on top of the LEDs matrix we recorded the output voltage signal provided by the Optical Power and Energy Meter PM400. The voltage signal can easily converted to the corresponding optical power using the equation: ${Optical}_{Power} \left(W\right) = \frac{{V}_{Out _ Console}}{2 V} ⋅ \frac{{I}_{full _ Scale}}{Respositivity \left(λ\right)}$ [1] Where *I_{full_scale}* can be evaluated depending on the amplification coefficient applied by the console on the raw voltage of the photodiode (see table below).

### Current input Phtodlode Sensors

| **Current range** | **Display Resolution** | **Measurement Accuracy** | **Analog Output Gain** | **Analog Output Bandwidth** |
|---|---|---|---|---|
| 5 mA | 1 µA | +/- 0.2% f.s. | 4 x 10² V/A | 100 kHz / 25 Hz |
| 500 µA | 100 nA | +/- 0.2% f.s. | 4 x 10³ V/A | 100 kHz / 25 Hz |
| 50 | 10 nA | +/- 0.2% f.s. | 4 x 10⁴ V/A | 100 kHz / 25 Hz |
| 5 µA | 1 nA | +1- 0.2% f.s. | 4 x 10⁵ V/A | 100 kHz / 25 Hz |
| 500 nA | 100 pA | +/- 0.2% f.s. | 4 x 10⁶ V/A | 10 kHz / 25 Hz |
| 50 nA | 10 pA. | *+*/*-* 0.5% f.s. | 4 x 10⁷ V/A | 1 kHz / 25 Hz |

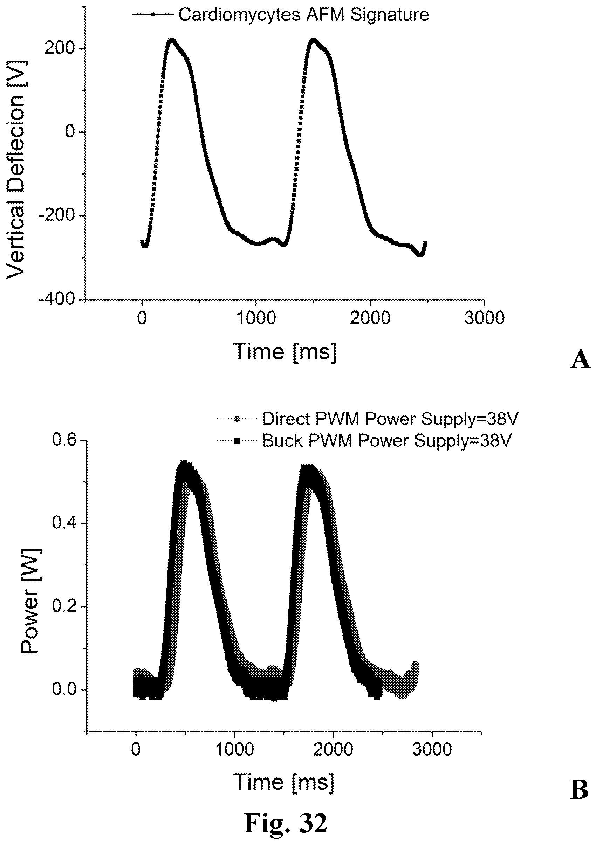

Thus, the buck and the direct PWM were compared with respect to the cardiomyocyte cell signature obtained from AFM curves, so as to track them as shown in the illustrative graphs of figures 32A and 32B. We can clearly see how the cardiomyocytes AFM signature is reproduced fairly well in both the buck and direct PWM case. When the direct PWM circuit is power supplied at his maximum voltage (44V), the LEDs matrix reaches temperatures higher than 37°C and the maximum optical power reaches approximatively 1 W. Thus, we decided to reduce the power supply to 38 V, in this case the temperature measured on top of the LEDs matrix is around 37°C. Finally, we compared the buck with respect to the direct PWM in the same power supply conditions.

Since the buck PWM provides a more stable optical power signal during time, maintaining a lower temperature on the LEDs matrix surface with respect the direct case, we decided to use the buck PWM circuit. Furthermore, the direct PWM stresses more the LEDs matrix since it continuously switched on/off the LED at the carrier frequency of the signal (1kHz).

The hardware architecture described above which refers to the signal modulation part, is integrated on a PCBA electronic board on which there are components for the management of the electric power, a communication and programming logic, a microprocessor and the output towards a connector that connects directly to the wearable.

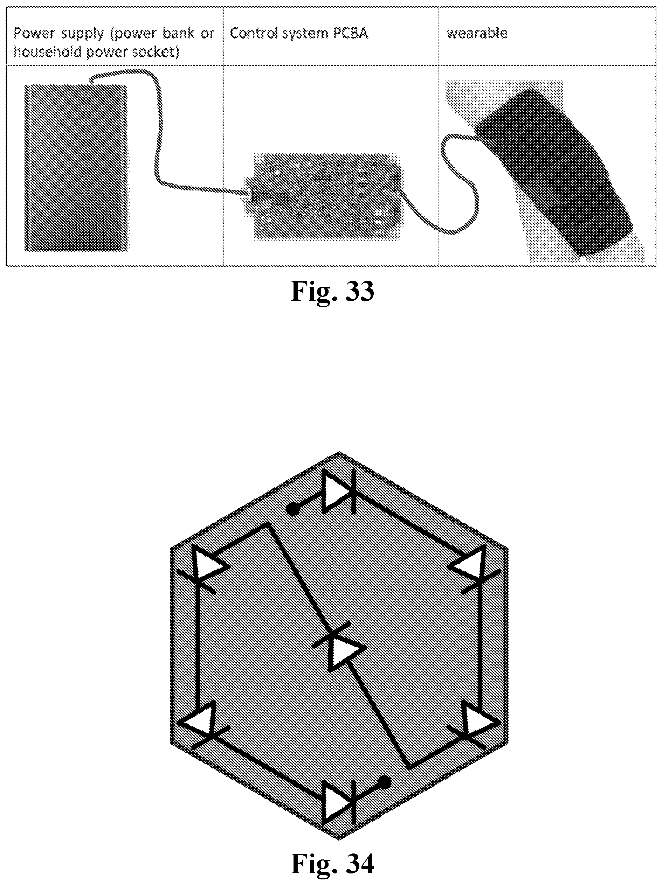

A sample embodiment of the wearable is made up as shown in Figure 33.

The wearable, which varies according to the area of the body where it is applied, consists of a specific electronic design.

Inside the wearable are individual PCBAs made up of hexagon shaped PCBAs containing 7 LEDs for each PCBA. The LEDs used are high power devices (1W), with wavelength of 810nm: WL-1P3030EP120IR-810.

Each of them is supplied by 1.7V (nominal) with 350mA (max), while for the operating conditions the table below is presented:

| **Characteristics** | **Unit of measurement** | **Performances** |
|---|---|---|
| Operating Temperature Range | °C | -25 ÷ 85 |
| Working temperature | °C | <50 |
| Storage Temperature Range | °C | -35 ÷ 100 |

The distance among the LEDs is between 10 and 20 mm, more preferably between 14 and 18 mm. The 7 LEDs are connected in series, generating 11.9 Volts and 0.35A.

The zone configuration consists, at most, of 2 parallel sets of 4 PCBAs generating 47.6 Volts and 0.7A for each zone, as shown in figure 34.

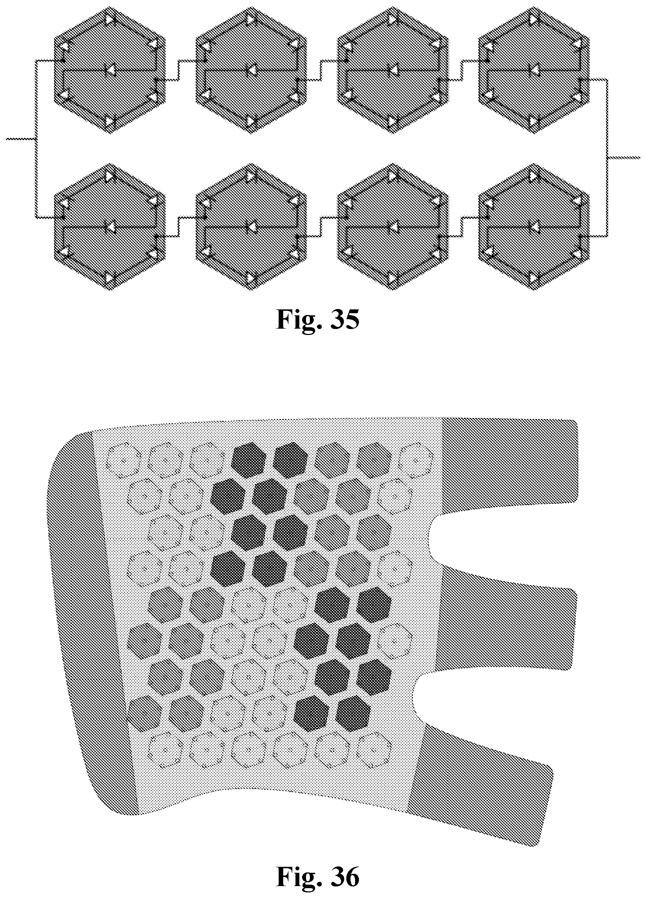

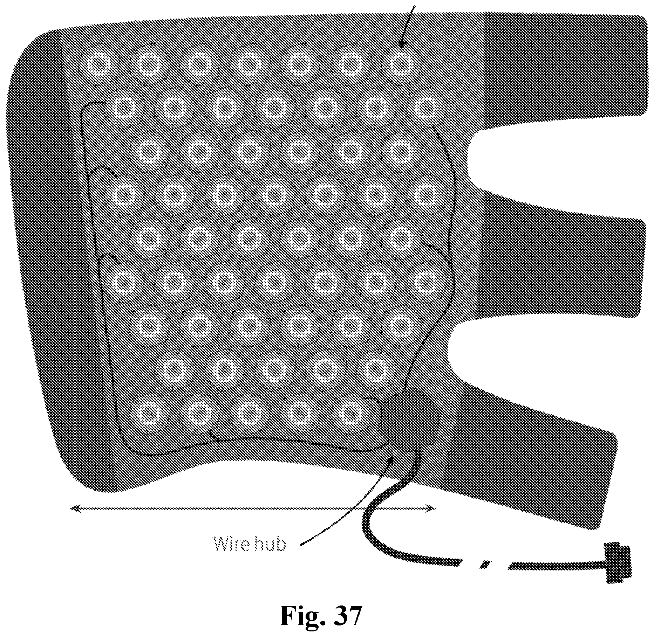

The distribution of the zones in the wearable can be varied depending on where the wearable is applied. According to an aspect, the distribution of the zones may be as shown in figure 35. The hexagon-shaped PCBA are distributed above the leg ensuring a distance between the LEDs of 14 mm from each other. The distribution, indicated as an example, leads to having 6 zones of 8 PCBAs each for a total of 48 PCBA and 336 LEDs, as shown by way of example in Figure 36. Each zone has its internal connections between the 8 PCBAs and is connected to an electrical collector which unites all the zones. The electrical collector is connected to a connector through an electrical cable, as shown in a non-limitative manner in Figure 37.

According to an aspect, each group of LEDs (or NIR-LEDs) of the plurality of group of LEDs of the actuator is allocated in a FPCB (flexible Printed Circuit Board) irradiation zone comprising a plurality of LEDs electrically in series (two series of LEDs in series connected together in parallel oriented vertically). The LEDs are spatially placed following an equilateral triangular pattern. According to an aspect, each irradiation zone accommodates 54 LEDs, and the actuator comprises 6 irradiation zones.

According to an aspect, the LEDs (or NIR-LEDs) are arranged according to a generic geometric pattern so as to be equally spaced on the Flexible Printed Circuit (FPC).

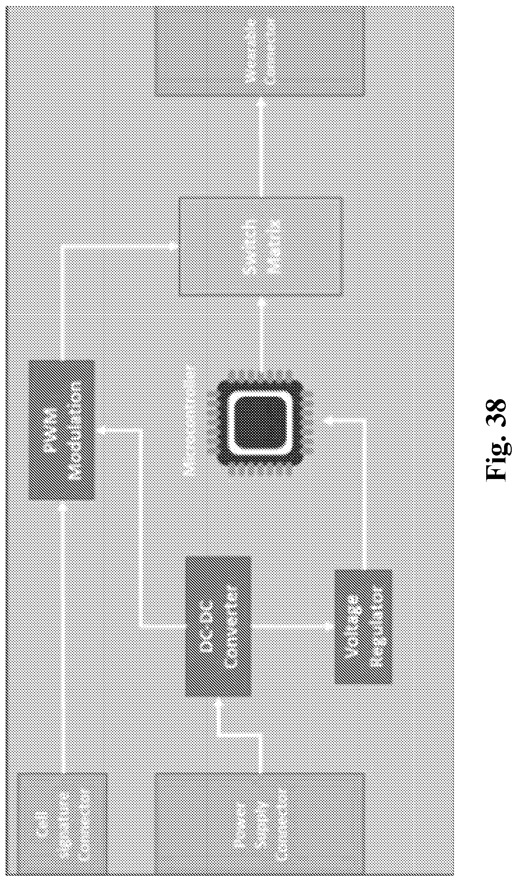

An illustrative embodiment of a control system for an appliance for stimulating a living human or animal cell, according to this disclosure, is shown in the scheme depicted in Figure 38. According to the shown illustrative embodiment, there may be the following main components:
CELL SIGNATURE CONNECTOR: to be able to load, modify and program the signals to be modulated during treatment through the wearable
POWER SUPPLY CONNECTOR: to connect the control system to a power bank or home network
DC-DC CONVERTER: to convert the voltage to the wearable
PWM MODULATION: to generate wave functions that faithfully report the signatures
VOLTAGE REGULATOR: for regulating the output voltage
MICROCONTROLLER: to manage the driving logic of the wearable
SWITCH MATRIX: to intervene on the different areas of the wearable
WEARABLE CONNECTOR: as an interface to the wearable.

The power supply can be portable or fixed. In the portable case, it consists of a power bank which must guarantee voltage and amperage to power the LEDs and the control system circuitry. In the domestic case it will be the 220 Volts domestic power outlet.

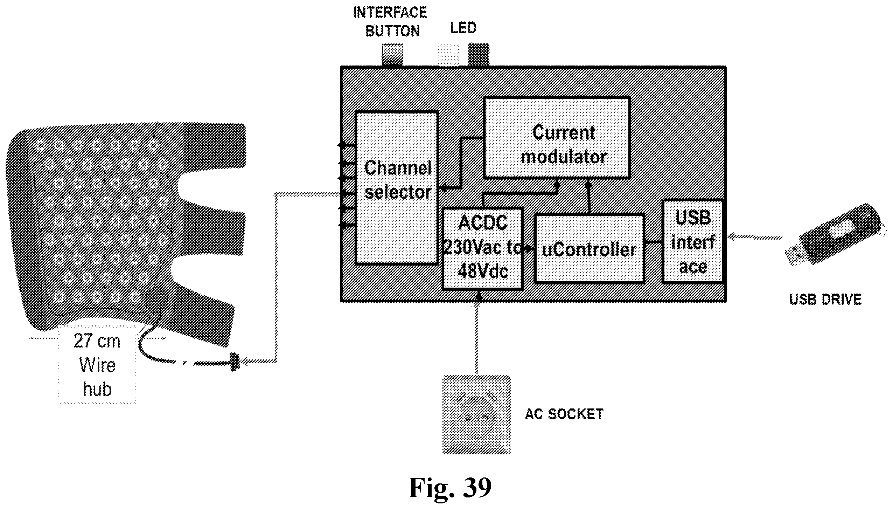

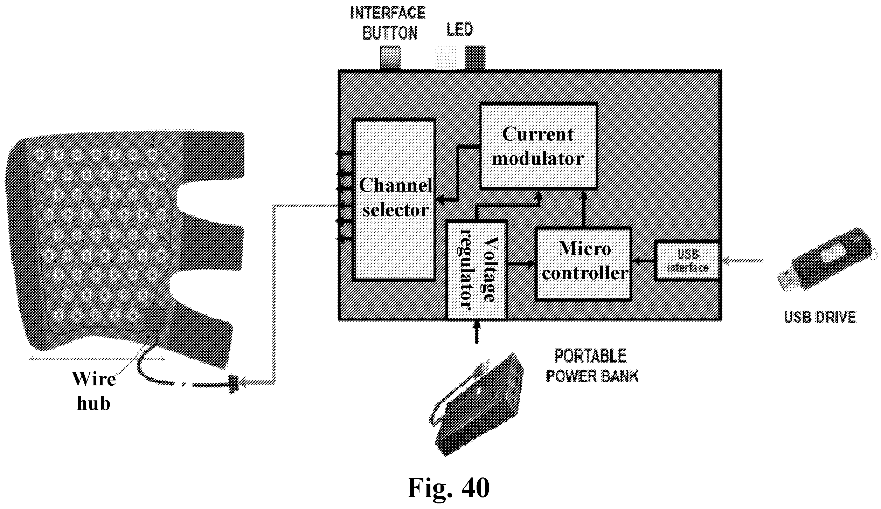

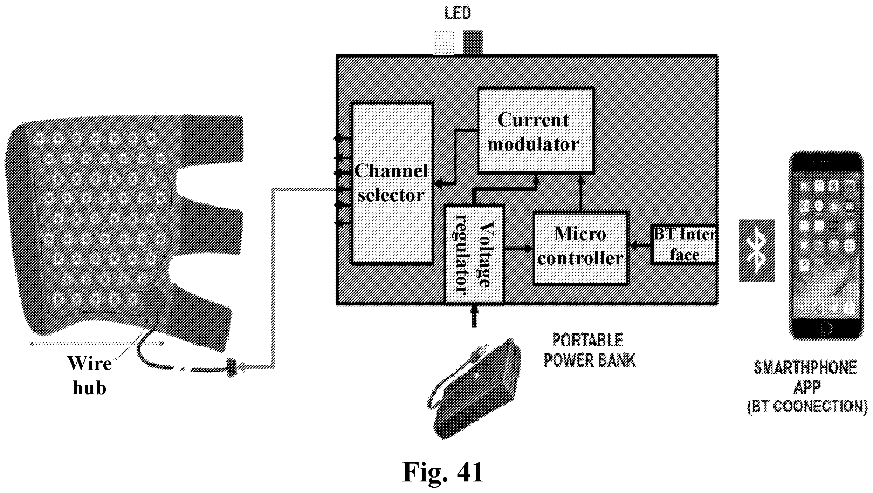

Other embodiments of an appliance for stimulating a living human or animal cell are shown in figures 39 to 41. Basically, all appliances comprise:
- a controlled piezo-electric or light actuator, configured to generate a mechanical or light stimulation to be applied to the living human or animal cell;
- a microprocessor unit, configured to receive data of a characteristic signal of the living human or animal cell and configured to generate corresponding command signals for the piezo-electric or light actuator so as to cause the piezo-electric or light actuator to generate the mechanical or light stimulation corresponding to the data of the characteristic signal. The data of the characteristic signal may be processed by the microprocessor unit in real time as soon as the characteristic signal is sensed, or more conveniently the data of the characteristic signal may be stored in a non-volatile memory, which may be accessed by the microprocessor unit, and downloaded by the microprocessor unit when the command signal for the piezo-electric or light actuator have to be generated.

In the shown illustrative embodiments, there is also a current modulator configured to be controlled by the microprocessor unit and to generate a waveform of a control voltage or control current corresponding to the data of the characteristic signal. The current modulator is functionally coupled to control the controlled piezo-electric or light actuator with the control voltage or control current to cause the piezo-electric or light actuator to generate the mechanical or light stimulation corresponding to the data of the characteristic signal. The actuator may comprise a plurality of groups of LEDs supplied by a respective plurality of electric lines, the LEDs of each group being connected electrically in series among them and configured to be supplied through a corresponding electric line of the electric lines. The appliance further comprises a channel selector controlled by the microprocessor unit through a command signal and connected to the current modulator and the actuator, configured to receive from the current modulator a current and to supply selectively the groups of LEDs through the electric lines depending on the command signal generated by the microprocessor unit.

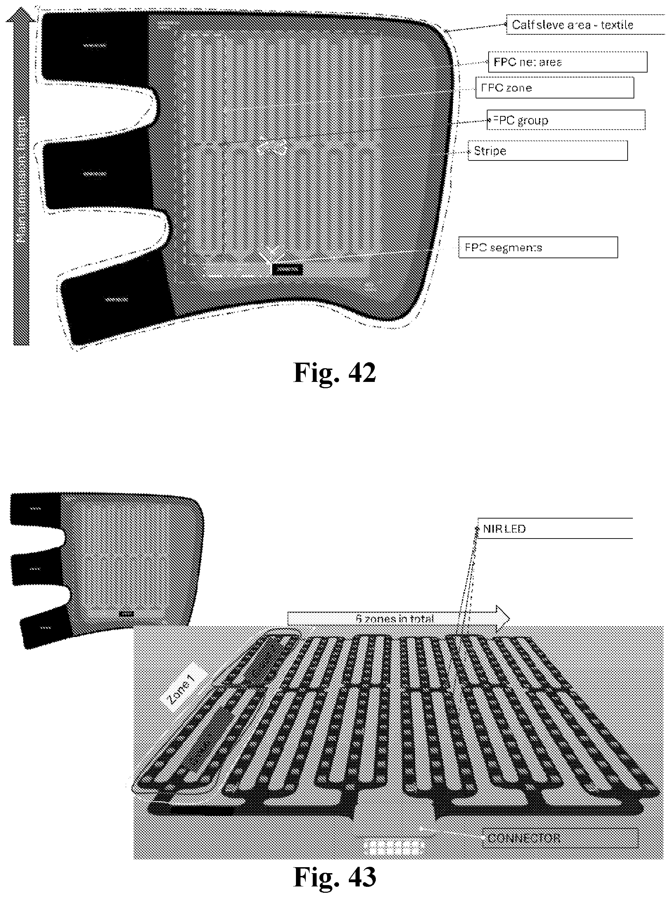

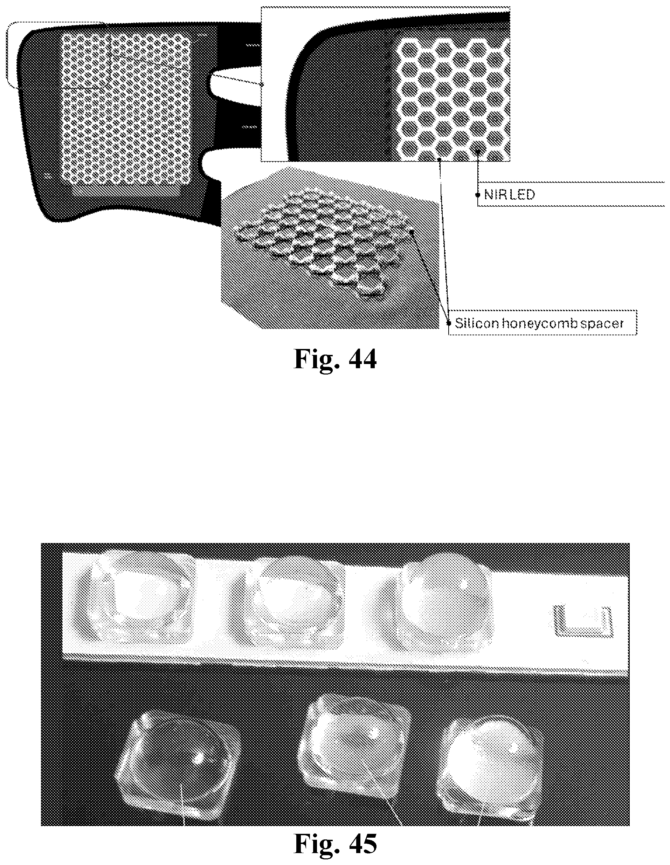

According to a sample embodiment, illustrated in figures 42-45, an appliance of the present disclosure may be realized as a wearable calf sleeve. In the shown example, the wearable calf sleeve embodies Near-InfraRed Light Emitting Diodes (NIR-LEDs) mounted on flexible pcb (namely FPC), the latter installed at the innermost side of the calf textile sleeve itlself and kept distant from the target through a silicon grid spacer, the whole structure covering most of the calf sleeve area.

With reference to figures 42-45, hereby there are recapped the main features of this sample embodiment. The FPC geometry comes in the shape of a net of stripes, the latter mainly parallel each other, which main direction follows the calf shape in its length. The net of stripes is completed by FPC segments, typically shorter than the stripes, segments which are spatially in continuity with the stripes, segments which deviate from the main direction and which purpose is to link the parallel stripes each other and to the supply.

The FPC is provided with NIR-LEDs mounted along its stripes; LEDs are preferably evenly distributed along each stripe so as to provide, in conjunction with a proper spacing among stripes and from LED source to the target, with an as much as possible uniform radiation to the target while operating.

The FPC with NIR-LEDs is divided into groups: each group embodies a proper number of stripes parallel each other and shorter segments. Groups can be replicated both vertically and circumferentially to form a zone; LEDs belonging to each group are electrically connected in series by conductive routes embedded in the FPC group stripes and segments. Groups of each zone are electrically connected in parallel.

Each zone is supplied through at least one connector, so that operating conditions can include switching-on one zone LEDs at a time, sequentially, or more then one zone LEDs at a time, sequentially or not. Intensity levels can be set arbitrarily for each zone.

NIR LEDs embedded upon FPC are kept at a given distance from the target surface by means of a silicone grid covering almost all the illuminating area, or partially. The silicone grid is placed directly on the textile and can be co-moulded or placed separately upon it. The form of such silicon spacer is such that no physical barriers are placed between LEDs source and the target.

The NIR-LED components include at least a protection such as a silicon glue or similar placed upon the emitting source. Upon each NIR-LED component there could a be optionally a lens which aim is both to protect the chip and to redirect radiation as per custom design.

As an example, hereby is it possible to look at the features of one specific product.
- Number of connectors: 1
- Number of Zones: 6
- Number of groups: 12 (2 each zone)
- Number of stripes: 36 (3 each groups)
- Number of segments: NA (they are all the fpc segments wich are not the vertical one where LEDS are mounted);
- Number of NIR LEDS: 324 (9 each stripe)
- Number of series-connected LEDs per group: 27
- Number of groups in parallel: 2
- Operation: one zone illuminated at a time, sequentially.
- Silicon spacer: silicon honeycomb pattern

### REFERENCES

1. Tassinari R, Cavallini C, Olivi E, Facchin F, Taglioli V, Zannini C, Marcuzzi M, Ventura C. Cell Responsiveness to Physical Energies: Paving the Way to Decipher a Morphogenetic Code. Int J Mol Sci. 2022, 23, 3157 [DOI: 10.3390/ijms23063157].
2. Segura-Valdez ML, Agredano-Moreno LT, Zamora-Cura AL, Lara-Martínez R, Jiménez-García LF. Visualization of internal in situ cell structure by atomic force microscopy. Histochem. Cell Biol. 2018, 150, 521-527 [DOI: 10.1007/s00418-018-1721-6]
3. Cosgrove DJ. Nanoscale structure, mechanics and growth of epidermal cell walls. Curr. Opin.Plant B iol. 2018, 46, 77-86 [DOI: 10.1016/j.pbi.2018.07.016]
4. Amarouch MY, El Hilaly J, Mazouzi D. AFM and FluidFM Technologies: Recent Applications in Molecular and Cellular Biology. Scanning 2018, 2018, 7801274 [DOI: 10.1155/2018/7801274]
5. Sharma S, LeClaire M, Gimzewski JK. Ascent of atomic force microscopy as a nanoanalytical tool for exosomes and other extracellular vesicles. Nanotechnology 2018, 29, 132001 [DOI: 10.1088/1361-6528/aaab06]
6. Sahu S, Ghosh S, Fujita D, Bandyopadhyay A. Live visualizations of single isolated tubulin protein self-assembly via tunneling current: effect of electromagnetic pumping during spontaneous growth of microtubule. Sci. Rep. 2014, 4, 7303 [DOI: 10.1038/srep07303]
7. Agrawal L, Sahu S, Ghosh S, Shiga T, Fujita D, Bandyopadhyay A. Inventing atomic resolution scanning dielectric microscopy to see a single protein complex operation live at resonance in a neuron without touching or adulterating the cell. J. Integr. Neurosci. 2016, 15, 435-462 [DOI: 10.1142/S0219635216500333]
8. Acbas G, Niessen KA, Snell EH, Markelz AG. Optical measurements of long-range protein vibrations. Nat. Commun. 2014, 5, 3076 [DOI: 10.1038/ncomms4076]
9. Cheng JX, Xie XS. Vibrational spectroscopic imaging of living systems: An emerging platform for biology and medicine. Science 2015, 350, aaa8870 [DOI: 10.1126/science.aaa8870]
10. Browne AW, Arnesano C, Harutyunyan N, Khuu T, Martinez JC, Pollack HA, Koos DS, Lee TC, Fraser SE, Moats RA, Aparicio JG, Cobrinik D. Structural and Functional Characterization of Human Stem-Cell-Derived Retinal Organoids by Live Imaging. Invest. Ophthalmol. Vis. Sci. 2017, 58, 3311-3318 [DOI: 10.1167/iovs.16-20796]
11. Heraud P, Cowan MF, Marzec KM, Møller BL, Blomstedt CK, Gleadow R. Label-free Raman hyperspectral imaging analysis localizes the cyanogenic glucoside dhurrin to the cytoplasm in sorghum cells. Sci. Rep. 2018, 8, 2691 [DOI: 10.1038/s41598-018-20928-7]
12. Blackshaw S, Snyder SH. Encephalopsin: a novel mammalian extraretinal opsin discretely localized in the brain. J. Neurosci. 1999, 19, 3681-3690 [DOI: 10.1523/JNEUROSCI.19-1003681.1999]
13. Hoang N, Schleicher E, Kacprzak S, Bouly JP, Picot M, Wu W, Berndt A, Wolf E, Bittl R, Ahmad M. Human and Drosophila cryptochromes are light activated by flavin photoreduction in living cells. PLoS Biol. 2008, 6, e160 [DOI: 10.1371/journal.pbio.0060160]
14. Koyanagi M, Terakita A. Diversity of animal opsin-based pigments and their optogenetic potential. Biochim. Biophys. Acta 2014, 1837, 710-716 [DOI: 10.1016/j.bbabio.2013.09.003]
15. Porter ML, Blasic JR, Bok MJ, Cameron EG, Pringle T, Cronin TW, Robinson PR. Shedding new light on opsin evolution. Proc. Biol. Sci. 2012, 279, 3-14 [DOI: 10. 1098/rspb.2011.1819]
16. Bailes HJ, Lucas RJ. Human melanopsin forms a pigment maximally sensitive to blue light (lambdamax approximately 479 nm) supporting activation of G(q/11) and G(i/o) signalling cascades. Proc. Biol. Sci. 2013, 280, 20122987 [DOI: 10.1098/rspb.2012.2987]
17. Ankri R, Friedman H, Savion N, Kotev-Emeth S, Breitbart H, Lubart R. Visible light induces nitric oxide (NO) formation in sperm and endothelial cells. Lasers Surg. Med. 2010, 42, 348-352 [DOI: 10.1002/lsm.20849]
18. Garza ZCF, Born M, Hilbers PAJ, van Riel NAW, Liebmann J. Visible Blue Light Therapy: Molecular Mechanisms and Therapeutic Opportunities. Curr. Med. Chem. 2018, 25, 5564-5577 [DOI: 10.2174/0929867324666170727112206]
19. Iyanagi T. Molecular mechanism of metabolic NAD(P)H-dependent electron-transfer systems: The role of redox cofactors. Biochim. Biophys. Acta Bioenerg. 2019, 1860, 233-258 [DOI: 10.1016/j.bbabio.2018.11.014]
20. Terakita A, Nagata T. Functional properties of opsins and their contribution to light-sensing physiology. Zoolog. Sci. 2014, 31, 653-659 [DOI: 10.2108/zs140094]
21. Kojima D, Mori S, Torii M, Wada A, Morishita R, Fukada Y. UV-sensitive photoreceptor protein OPN5 in humans and mice. PLoS One 2011, 6, e26388 [DOI: 10.1371/journal.pone.0026388]
22. Wang L, Zhang D, Schwarz W. TRPV Channels in Mast Cells as a Target for Low-Level-Laser Therapy. Cells 2014, 3, 662-673 [DOI: 10.3390/cells3030662]
23. Wu ZH, Zhou Y, Chen JY, Zhou LW. Mitochondrial signaling for histamine releases in laserirradiated RBL-2H3 mast cells. Lasers Surg. Med. 2010, 42, 503-509 [DOI: 10.1002/lsm.20924]
24. Yang WZ, Chen JY, Yu JT, Zhou LW. Effects of low power laser irradiation on intracellular calcium and histamine release in RBL-2H3 mast cells. Photochem. Photobiol. 2007, 83, 979-984 [DOI: 10.1111/j.1751-1097.2007.00116.x]
25. Kumbalasiri T, Provencio I. Melanopsin and other novel mammalian opsins. Exp. Eye Res. 2005, 81, 368-375 [DOI: 10.1016/j.exer.2005.05.004]
26. Terakita A. The opsins. Genome Biol. 2005, 6, 213 [DOI: 10.1186/gb-2005-6-3-213]
27. Sikka G, Hussmann GP, Pandey D, Cao S, Hori D, Park JT, Steppan J, Kim JH, Barodka V, Myers AC, Santhanam L, Nyhan D, Halushka MK, Koehler RC, Snyder SH, Shimoda LA, Berkowitz DE. Melanopsin mediates light-dependent relaxation in blood vessels. Proc Natl Acad Sci U S A 2014, 111, 17977-17982 [DOI: 10.1073/pnas.1420258111]
28. Barreto Ortiz S, Hori D, Nomura Y, Yun X, Jiang H, Yong H, Chen J, Paek S, Pandey D, Sikka G, Bhatta A, Gillard A, Steppan J, Kim JH, Adachi H, Barodka VM, Romer L, An SS, Shimoda LA, Santhanam L, Berkowitz DE. Opsin 3 and 4 mediate light-induced pulmonary vasorelaxation that is potentiated by G protein-coupled receptor kinase 2 inhibition. Am J Physiol Lung Cell Mol Physiol. 2018, 314, L93-L106 [DOI: 10.1152/ajplung.00091.2017]
29. Stachurska A, Sarna T. Regulation of Melanopsin Signaling: Key Interactions of the Nonvisual Photopigment. Photochem Photobiol. 2019, 95, 83-94 [DOI: 10.1111/php.12995]
30. Sahu S, Ghosh S, Hirata K, Fujita D, Bandyopadhyay A. Multi-level memory-switching properties of a single brain microtubule. Appl. Phys. Lett. 2013, 102, 123701 [DOI: 10.1063/1.4793995]
31. Celardo GL, Angeli M, Craddock TJA, Kurian P. On the existence of superradiant excitonic states in microtubules. New J. Phys. 2019, 21, 023005 [DOI: 10.1088/1367-2630/aaf839]
32. O'Keeffe KP, Hong H, Strogatz SH. Oscillators that sync and swarm. Nat. Commun. 2017, 8, 1504 [DOI: 10.1038/s41467-017-01190-3]
33. Sumino Y, Nagai KH, Shitaka Y, Tanaka D, Yoshikawa K, Chaté H, Oiwa K. Large-scale vortex lattice emerging from collectively moving microtubules. Nature 2012, 483, 448-452 [DOI: 10.1038/nature10874]
34. Kim HB, Baik KY, Choung PH, Chung JH. Pulse frequency dependency of photobiomodulation on the bioenergetic functions of human dental pulp stem cells. Sci. Rep. 2017, 7, 15927 [DOI: 10.1038/s41598-017-15754-2]
35. Zomorrodi R, Loheswaran G, Pushparaj A, Lim L. Pulsed Near Infrared Transcranial and Intranasal Photobiomodulation Significantly Modulates Neural Oscillations: a pilot exploratory study. Sci. Rep. 2019, 9, 6309 [DOI: 10.1038/s41598-019-42693-x]
36. Iaccarino HF, Singer AC, Martorell AJ, Rudenko A, Gao F, Gillingham TZ, Mathys H, Seo J, Kritskiy O, Abdurrob F, Adaikkan C, Canter RG, Rueda R, Brown EN, Boyden ES, Tsai LH. Gamma frequency entrainment attenuates amyloid load and modifies microglia. Nature 2016, 540, 230235 [DOI: 10.1038/nature20587]
37. Martorell AJ, Paulson AL, Suk HJ, Abdurrob F, Drummond GT, Guan W, Young JZ, Kim DN, Kritskiy O, Barker SJ, Mangena V, Prince SM, Brown EN, Chung K, Boyden ES, Singer AC, Tsai LH. Multisensory Gamma Stimulation Ameliorates Alzheimer's-Associated Pathology and Improves Cognition. Cell 2019, 177, 256-271.e22 [DOI: 10.1016/j.cell.2019.02.014]
38. Zheng L, Yu M, Lin R, Wang Y, Zhuo Z, Cheng N, Wang M, Tang Y, Wang L, Hou ST. Rhythmic light flicker rescues hippocampal low gamma and protects ischemic neurons by enhancing presynaptic plasticity. Nat. Commun. 2020, 11, 3012 [DOI: 10.1038/s41467-02016826-0]
39. Maioli M, Rinaldi S, Santaniello S, Castagna A, Pigliaru G, Gualini S, Fontani V, Ventura C. Radio frequency energy loop primes cardiac, neuronal, and skeletal muscle differentiation in mouse embryonic stem cells: a new tool for improving tissue regeneration. Cell Transplant. 2012, 21, 1225-1233 [DOI: 10.3727/096368911X600966]
40. Maioli M, Rinaldi S, Santaniello S, Castagna A, Pigliaru G, Delitala A, Bianchi F, Tremolada C, Fontani V, Ventura C. Radio electric asymmetric conveyed fields and human adipose-derived stem cells obtained with a non-enzymatic method and device: a novel approach to multipotency. Cell Transplant. 2014, 23, 1489-1500 [DOI: 10.3727/096368913X672037]
41. Ventura C, Maioli M, Pintus G, Gottardi G, Bersani F. Elf-pulsed magnetic fields modulate opioid peptide gene expression in myocardial cells. Cardiovasc. Res. 2000, 45, 1054-1064 [DOI: 10.1016/S0008-6363(99)00408-3]
42. Ventura C, Maioli M, Asara Y, Santoni D, Mesirca P, Remondini D, Bersani F. Turning on stem cell cardiogenesis with extremely low frequency magnetic fields. FASEB J. 2005, 19, 155-157 [DOI: 10.1096/fj.04-2695fje]
43. Maioli M, Rinaldi S, Santaniello S, Castagna A, Pigliaru G, Gualini S, Cavallini C, Fontani V, Ventura C. Radio electric conveyed fields directly reprogram human dermal-skin fibroblasts toward cardiac-, neuronal-, and skeletal muscle-like lineages. Cell Transplant. 2013, 22, 1227-1235 [DOI: 10.3727/096368912X657297]
44. Rinaldi S, Maioli M, Pigliaru G, Castagna A, Santaniello S, Basoli V, Fontani V, Ventura C. Stem cell senescence. Effects of REAC technology on telomerase-independent and telomerasedependent pathways. Sci. Rep. 2014, 4, 6373 [DOI: 10.1038/srep06373]
45. Maioli M, Rinaldi S, Pigliaru G, Santaniello S, Basoli V, Castagna A, Fontani V, Ventura C. REAC technology and hyaluron synthase 2, an interesting network to slow down stem cell senescence. Sci. Rep. 2016, 6, 28682 [10.1038/srep28682]
46. Maioli M, Rinaldi S, Migheli R, Pigliaru G, Rocchitta G, Santaniello S, Basoli V, Castagna A, Fontani V, Ventura C, Serra PA. Neurological morphofunctional differentiation induced by REAC technology in PC12. A neuro protective model for Parkinson's disease. Sci. Rep. 2015, 5, 10439 [DOI: 10.1038/srep10439]
47. Tassinari R, Olivi E, Cavallini C, Taglioli V, Zannini C, Marcuzzi M, Fedchenko O, Ventura C. Mechanobiology: A landscape for reinterpreting stem cell heterogeneity and regenerative potential in diseased tissues. iScience. 2022, 26,105875 [DOI: 10.1016/j.isci.2022.105875] eCollection 2023 Jan 20.PMID: 36647385
48. Pandur P, Sirbu IO, Kühl SJ, Philipp M, Kühl M. Islet1-expressing cardiac progenitor cells: a comparison across species. Dev Genes Evol. 2013, 223, 117-129 [DOI: 10.1007/s00427-0120400-1]
49. Fonoudi H, Yeganeh M, Fattahi F, Ghazizadeh Z, Rassouli H, Alikhani M, Mojarad BA, Baharvand H, Salekdeh GH, Aghdami N. ISL1 protein transduction promotes cardiomyocyte differentiation from human embryonic stem cells. PLoS One. 2013, 8, e55577 [DOI: 10.1371/journal.pone.0055577]
50. Canac R, Cimarosti B, Girardeau A, Forest V, Olchesqui P, Poschmann J, Redon R, Lemarchand P, Gaborit N, Lamirault G. Deciphering Transcriptional Networks during Human Cardiac Development. Cells. 2022, 11, 3915 [DOI: 10.3390/cells11233915]
51. Mauritz C, Schwanke K, Reppel M, Neef S, Katsirntaki K, Maier LS, Nguemo F, Menke S, Haustein M, Hescheler J, Hasenfuss G, Martin U. Generation of functional murine cardiac myocytes from induced pluripotent stem cells. Circulation. 2008, 118, :507-517 [DOI: 10.1161/CIRCULATIONAHA.108.778795]
52. Hong GS, Lee SH, Lee B, Choi JH, Oh SJ, Jang Y, Hwang EM, Kim H, Jung J, Kim IB, Oh U. ANO1/TMEM16A regulates process maturation in radial glial cells in the developing brain. Proc Natl Acad Sci U S A. 2019, 116, 12494-12499 [DOI: 10.1073/pnas.1901067116]
53. Der Vartanian A, Quétin M, Michineau S, Auradé F, Hayashi S, Dubois C, Rocancourt D, DraytonLibotte B, Szegedi A, Buckingham M, Conway SJ, Gervais M, Relaix F. PAX3 Confers Functional Heterogeneity in Skeletal Muscle Stem Cell Responses to Environmental Stress. Cell Stem Cell. 2019, 24, 958-973.e9 [DOI: 10.1016/j.stem.2019.03.019]
54. Korzh V, Strähle U. Proneural, prosensory, antiglial: the many faces of neurogenins. Trends Neurosci. 2002, 25, 603-605 [DOI: 10.1016/s0166-2236(02)02275-0]
55. Gao F, Wei Z, An W, Wang K, Lu W. The interactomes of POUSF1 and SOX2 enhancers in human embryonic stem cells. Sci Rep. 2013, 3, 1588 [DOI: 10.1038/srep01588]
56. Ovchinnikov DA, Titmarsh DM, Fortuna PR, Hidalgo A, Alharbi S, Whitworth DJ, Cooper-White JJ, Wolvetang EJ. Transgenic human ES and iPS reporter cell lines for identification and selection of pluripotent stem cells in vitro. Stem Cell Res. 2014, 13, 251-261 [DOI: 10.1016/j.scr.2014.05.006]
57. Ramirez JM, Gerbal-Chaloin S, Milhavet O, Qiang B, Becker F, Assou S, Lemaitre JM, Hamamah S, De Vos J. Brief report: benchmarking human pluripotent stem cell markers during differentiation into the three germ layers unveils a striking heterogeneity: all markers are not equal. Stem Cells. 2011, 29, 1469-1474 [DOI: 10.1002/stem.681]
58. Zwi-Dantsis L, Huber I, Habib M, Winterstern A, Gepstein A, Arbel G, Gepstein L. Derivation and cardiomyocyte differentiation of induced pluripotent stem cells from heart failure patients. Eur Heart J. 2013, 34, 1575-1586 [DOI: 10.1093/eurheartj/ehs096]
59. Peterkin T, Gibson A, Patient R. GATA-6 maintains BMP-4 and Nkx2 expression during cardiomyocyte precursor maturation. EMBO J. 2003, 22, 4260-4273 [DOI: 10.1093/emboj/cdg400]
60. Hu Y, Wang Q, Yu J, Zhou Q, Deng Y, Liu J, Zhang L, Xu Y, Xiong W, Wang Y. Tartrate-resistant acid phosphatase 5 promotes pulmonary fibrosis by modulating β-catenin signaling. Nat Commun. 2022, 13, 114 [DOI: 10.1038/s41467-021-27684-9]
61. Kania G, Blyszczuk P, Stein S, Valaperti A, Germano D, Dirnhofer S, Hunziker L, Matter CM, Eriksson U. Heart-infiltrating prominin-1+/CD133+ progenitor cells represent the cellular source of transforming growth factor beta-mediated cardiac fibrosis in experimental autoimmune myocarditis. Circ Res. 2009, 105, 462-470 [DOI: 10.1161/CIRCRESAHA.109.196287]
62. Blyszczuk P, Miiller-Edenborn B, Valenta T, Osto E, Stellato M, Behnke S, Glatz K, Basler K, Liischer TF, Distler O, Eriksson U, Kania G. Transforming growth factor-β-dependent Wnt secretion controls myofibroblast formation and myocardial fibrosis progression in experimental autoimmune myocarditis. Eur Heart J. 2017, 38, 1413-1425 [DOI: 10.1093/eurheartj/ehw116]
63. Czepiel M, Diviani D, Jaźwa-Kusior A, Tkacz K, Rolski F, Smolenski RT, Siedlar M, Eriksson U, Kania G, B 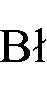 yszczuk P. Angiotensin II receptor 1 controls profibrotic Wnt/β-catenin signalling in experimental autoimmune myocarditis. Cardiovasc Res. 2022, 118, 573-584 [DOI: 10.1093/cvr/cvab039]
64. Yin H, Zhang J, Lin H, Wang R, Qiao Y, Wang B, Liu F. p38 mitogen-activated protein kinase inhibition decreases TNFalpha secretion and protects against left ventricular remodeling in rats with myocardial ischemia. Inflammation. 2008, 31, 65-73 [DOI: 10.1007/s10753-007-9050-2]
65. Arabacilar P, Marber M. The case for inhibiting p38 mitogen-activated protein kinase in heart failure. Front Pharmacol. 2015, 6, 102 [DOI: 10.3389/fphar.2015.00102]
66. Li Y, Li Z, Zhang C, Li P, Wu Y, Wang C, Bond Lau W, Ma XL, Du J. Cardiac Fibroblast-Specific Activating Transcription Factor 3 Protects Against Heart Failure by Suppressing MAP2K3-p38 Signaling. Circulation. 2017, 135, 2041-2057 [DOI: 10.1161/CIRCULATIONAHA.116.024599]
67. Stratton MS, Bagchi RA, Felisbino MB, Hirsch RA, Smith HE, Riching AS, Enyart BY, Koch KA, Cavasin MA, Alexanian M, Song K, Qi J, Lemieux ME, Srivastava D, Lam MPY, Haldar SM, Lin CY, McKinsey TA. Dynamic Chromatin Targeting of BRD4 Stimulates Cardiac Fibroblast Activation. Circ Res. 2019, 125, 662-677 [DOI: 10.1161/CIRCRESAHA.119.315125]
68. Fu X, Khalil H, Kanisicak O, Boyer JG, Vagnozzi RJ, Maliken BD, Sargent MA, Prasad V, ValienteAlandi I, Blaxall BC, Molkentin JD. Specialized fibroblast differentiated states underlie scar formation in the infarcted mouse heart. J Clin Invest. 2018, 128, 2127-2143 [DOI: 10.1172/JCI98215]
69. Hume RD, Kanagalingam S, Deshmukh T, Chen S, Mithieux SM, Rashid FN, Roohani I, Lu J, Doan T, Graham D, Clayton ZE, Slaughter E, Kizana E, Stempien-Otero AS, Brown P, Thomas L, Weiss AS, Chong JJH. Tropoelastin Improves Post-Infarct Cardiac Function. Circ Res. 2023, 132, 72-86 [DOI: 10.1161/CIRCRESAHA.122.321123]
70. Vukicevic S, Colliva A, Kufner V, Martinelli V, Moimas S, Vodret S, Rumenovic V, Milosevic M, Brkljacic B, Delic-Brkljacic D, Correa R, Giacca M, Maglione M, Bordukalo-Niksic T, Dumic-Cule I, Zacchigna S. Bone morphogenetic protein 1.3 inhibition decreases scar formation and supports cardiomyocyte survival after myocardial infarction. Nat Commun. 2022, 13, 81 [DOI: 10.1038/s41467-021-27622-9]
71. Williams C, Quinn KP, Georgakoudi I, Black LD 3rd. Young developmental age cardiac extracellular matrix promotes the expansion of neonatal cardiomyocytes in vitro. Acta Biomater. 2014, 10, 194-204 [DOI: 10.1016/j.actbio.2013.08.037]
72. Konstandin MH, Toko H, Gastelum GM, Quijada P, De La Torre A, Quintana M, Collins B, Din S, Avitabile D, Völkers M, Gude N, Fässler R, Sussman MA. Fibronectin is essential for reparative cardiac progenitor cell response after myocardial infarction. Circ Res. 2013, 113, 115-125 [DOI: 10.1161/CIRCRESAHA.113.301152]
73. Sa S, Wong L, McCloskey KE. Combinatorial fibronectin and laminin signaling promote highly efficient cardiac differentiation of human embryonic stem cells. Biores Open Access. 2014, 3, 150-161 [DOI: 10.1089/biores.2014.0018]
74. Zhao YT, Wang J, Yano N, Zhang LX, Wang H, Zhang S, Qin G, Dubielecka PM, Zhuang S, Liu PY, Chin YE, Zhao TC. Irisin promotes cardiac progenitor cell-induced myocardial repair and functional improvement in infarcted heart. J Cell Physiol. 2019, 234, 1671-1681 [DOI: 10.1002/jcp.27037]
75. D'Uva G, Aharonov A, Lauriola M, Kain D, Yahalom-Ronen Y, Carvalho S, Weisinger K, Bassat E, Rajchman D, Yifa O, Lysenko M, Konfino T, Hegesh J, Brenner O, Neeman M, Yarden Y, Leor J, Sarig R, Harvey RP, Tzahor E. ERBB2 triggers mammalian heart regeneration by promoting cardiomyocyte dedifferentiation and proliferation. Nat Cell Biol. 2015, 17, 627-638 DOI: 10.1038/ncb3149]
76. Pianca N, Sacchi F, Umansky KB, Chirivì M, Iommarini L, Da Pra S, Papa V, Bongiovanni C, Miano C, Pontis F, Braga L, Tassinari R, Pantano E, Patnala RS, Mazzeschi M, Cenacchi G, Porcelli AM, Lauriola M, Ventura C, Giacca M, Rizzi R, Tzahor E, D'Uva G. Glucocorticoid receptor antagonization propels endogenous cardiomyocyte proliferation and cardiac regeneration. Nat Cardiovasc Res. 2022, 1, 617-633 [DOI: 10.1038/s44161-022-00090-0]
77. Bjerre M, Pedersen SH, Møgelvang R, Lindberg S, Jensen JS, Galatius S, Flyvbjerg A. High osteopontin levels predict long-term outcome after STEMI and primary percutaneous coronary intervention. Eur J Prev Cardiol. 2013, 20, 922-929 [DOI: 10.1177/2047487313487083]
78. Tang VW. Collagen, stiffness, and adhesion: the evolutionary basis of vertebrate mechanobiology. Mol Biol Cell. 2020, 31, 1823-1834 [DOI: 10.1091/mbc]
79. Rusu M, Hilse K, Schuh A, Martin L, Slabu I, Stoppe C, Liehn EA. Biomechanical assessment of remote and postinfarction scar remodeling following myocardial infarction. Sci Rep. 2019, 9, 16744 [DOI: 10.1038/s41598-019-53351-7]
80. Weber KT. Cardiac interstitium in health and disease: the fibrillar collagen network. J Am Coll Cardiol. 1989, 13, 1637-1652 [DOI: 10.1016/0735-1097(89)90360-4]
81. Weber KT, Janicki JS, Shroff SG, Pick R, Chen RM, Bashey RI. Collagen remodeling of the pressureoverloaded, hypertrophied nonhuman primate myocardium. Circ Res. 1988, 62, 757-765 [DOI: 10.1161/01.res.62.4.757]
82. Lionetti V, Cantoni S, Cavallini C, Bianchi F, Valente S, Frascari I, Olivi E, Aquaro GD, Bonavita F, Scarlata I, Maioli M, Vaccari V, Tassinari R, Bartoli A, Recchia FA, Pasquinelli G, Ventura C. Hyaluronan mixed esters of butyric and retinoic acid affording myocardial survival and repair without stem cell transplantation. J Biol Chem. 2010, 285, 9949-9961 [DOI: 10.1074/jbc.M109.087254]
83. Li DS, Avazmohammadi R, Rodell CB, Hsu EW, Burdick JA, Gorman JH 3rd, Gorman RC, Sacks MS. How hydrogel inclusions modulate the local mechanical response in early and fully formed postinfarcted myocardium. Acta Biomater. 2020, 114, 296-306 [DOI: 10.1016/j.actbio.2020.07.046]
84. Missinato MA, Tobita K, Romano N, Carroll JA, Tsang M. Extracellular component hyaluronic acid and its receptor Hmmr are required for epicardial EMT during heart regeneration. Cardiovasc Res. 2015, 107, 487-98 [DOI: 10.1093/cvr/cvv190]
85. Sun J, Peterson EA, Wang AZ, Ou J, Smith KE, Poss KD, Wang J. hapln1 Defines an Epicardial Cell Subpopulation Required for Cardiomyocyte Expansion During Heart Morphogenesis and Regeneration. Circulation. 2022, 146, 48-63 [DOI: 10.1161/CIRCULATIONAHA.121.055468]
86. Petz A, Grandoch M, Gorski DJ, Abrams M, Piroth M, Schneckmann R, Homann S, Müller J, Hartwig S, Lehr S, Yamaguchi Y, Wight TN, Gorressen S, Ding Z, Kötter S, Krüger M, Heinen A, Kelm M, Gödecke A, Flögel U, Fischer JW. Cardiac Hyaluronan Synthesis Is Critically Involved in the Cardiac Macrophage Response and Promotes Healing After Ischemia Reperfusion Injury. Circ Res. 2019, 124, 1433-1447 [DOI: 10.1161/CIRCRESAHA.118.31328]
87. Wang N, Liu C, Wang X, He T, Li L, Liang X, Wang L, Song L, Wei Y, Wu Q, Gong C. Hyaluronic Acid Oligosaccharides Improve Myocardial Function Reconstruction and Angiogenesis against Myocardial Infarction by Regulation of Macrophages. Theranostics. 2019, 9, 1980-1992 [DOI: 10.7150/thno.31073]
88. Le LV, Mohindra P, Fang Q, Sievers RE, Mkrtschjan MA, Solis C, Safranek CW, Russell B, Lee RJ, Desai TA. Injectable hyaluronic acid based microrods provide local micromechanical and biochemical cues to attenuate cardiac fibrosis after myocardial infarction. Biomaterials. 2018, 169, 11-21 [DOI: 10.1016/j.biomaterials.2018.03.042]
89. Yu J, Seldin MM, Fu K, Li S, Lam L, Wang P, Wang Y, Huang D, Nguyen TL, Wei B, Kulkarni RP, Di Carlo D, Teitell M, Pellegrini M, Lusis AJ, Deb A. Topological Arrangement of Cardiac Fibroblasts Regulates Cellular Plasticity. Circ Res. 2018, 123, 73-85 [DOI: 10.1161/CIRCRESAHA.118.312589]
90. Pesce M, Duda GN, Forte G, Girao H, Raya A, Roca-Cusachs P, Sluijter JPG, Tschöpe C, Van Linthout S. Cardiac fibroblasts and mechanosensation in heart development, health and disease. Nat Rev Cardiol. 2022, Nov 14 [DOI: 10.1038/s41569-022-00799-2]

## Claims

1. A method of stimulating a living human or animal cell either in vivo or in vitro extracted from a human or animal body, comprising the following steps:
generating a characteristic signal of the living human or animal cell;
generating a mechanical stimulation or a modulated light stimulation by means of a piezo-electric or light actuator controlled according to data of the characteristic signal of said living human or animal cell, wherein said modulated light stimulation is generated by modulating with said characteristic signal an intensity of a light beam absorbable by said living human or animal cell;
applying said mechanical or light stimulation to said living human or animal cell;
wherein said characteristic signal is generated through the following operations:
placing the extracted living cell into a test solution;
sensing a vibration of a superficial portion of an external surface of said extracted living cell;
generating a transduced time-varying signal corresponding to said sensed vibration;
low-pass filtering values of said transduced time-varying signal in the frequency range below 20Hz, generating data of said characteristic signal as a low-pass filtered replica of the values of said transduced time-varying signal.

2. The method of claim 1, wherein said transduced signal is generated by:
procuring and installing an atomic force microscope having a sensing tip;
placing the sensing tip in contact with the external surface of the extracted living cell;
generating said transduced signal as an electric signal representative of vibrations sensed by said sensing tip.

3. The method of claim 1, wherein said transduced signal is generated by:
procuring and installing an illuminating laser configured to illuminate the external surface of the cell;
procuring and installing an optical sensor configured to sense light scattered by the external surface of the cell;
generating said transduced signal as an electric signal representative of intensity of light, received by the optical sensor, which has been scattered by said external surface.

4. The method according to one of the preceding claims, wherein said characteristic signal is generated as a low-pass filtered replica of values of said transduced signal in the frequency range below 10Hz, preferably below 8Hz.

5. An appliance for stimulating a living human or animal cell either in vivo or in vitro extracted from a human or animal body, comprising:
a controlled piezo-electric or light actuator, configured to generate a mechanical or light stimulation to be applied to the living human or animal cell;
a microprocessor unit, configured to receive data of a characteristic signal of said living human or animal cell generated through the following operations:
placing the extracted living cell into a test solution,
sensing a vibration of a superficial portion of an external surface of said extracted living cell,
generating a transduced time-varying signal corresponding to said sensed vibration,
low-pass filtering values of said transduced time-varying signal in the frequency range below 20Hz, generating data of said characteristic signal as a low-pass filtered replica of the values of said transduced time-varying signal;
said microprocessor unit being configured to generate corresponding command signals for the piezo-electric or light actuator so as to cause the piezo-electric or light actuator to generate the mechanical or light stimulation corresponding to the data of the characteristic signal.

6. The appliance of claim 5, further comprising a non-volatile memory storing said data of the characteristic signal, wherein said microprocessor is configured to download said data of the characteristic signal from the non-volatile memory and to process the data when the command signals for the piezo-electric or light actuator have to be generated.

7. The appliance of claim 5 or 6, further comprising a current modulator configured to be controlled by the microprocessor unit and to generate a waveform of a control voltage or control current corresponding to said data of the characteristic signal, wherein said current modulator is functionally coupled to control said controlled piezo-electric or light actuator with said control voltage or control current to cause the piezo-electric or light actuator to generate the mechanical or light stimulation corresponding to the data of the characteristic signal.

8. The appliance of claim 7, wherein:
said actuator comprises a plurality of groups of LEDs supplied by a respective plurality of electric lines, the LEDs of each group being connected electrically in series among them and configured to be supplied through a corresponding electric line of said electric lines;
the appliance further comprises a channel selector controlled by the microprocessor unit through a command signal and connected to the current modulator and said actuator, configured to receive from said current modulator a current and to supply selectively the groups of LEDs through said electric lines depending on said command signal generated by the microprocessor unit.

9. The appliance of claim 8, wherein each group of LEDs of said plurality of groups of LEDs of the actuator is allocated in a corresponding hexagonal irradiation zone of a Printed Circuit Board and comprises a plurality of LEDs electrically in series among them disposed along a perimeter and at a center of the corresponding hexagonal irradiation zone.

10. The appliance of claim 8, wherein each group of LEDs of said plurality of group of LEDs of the actuator is allocated in a FPCB (Flexible Printed Circuit Board) irradiation zone comprising a plurality of LEDs electrically in series, wherein the LEDs are spatially placed following an equilateral triangular pattern.

11. The appliance of claim 8 or 9, wherein said actuator is shaped as a wearable band that may be worn directly in contact with a skin of a user.

12. The appliance of claim 8 or 9, wherein said actuator is shaped in the form of a helmet/cup that may be worn directly in contact with the scalp, or said actuator is shaped in the form of a bite to be placed inside the mouth of a user in contact with the gingival mucosa and/or teeth of the user.

## Patentansprüche

1. Verfahren zum Stimulieren einer lebenden menschlichen oder tierischen Zelle entweder in vivo oder in vitro, die aus einem menschlichen oder tierischen Körper extrahiert wurde, umfassend die folgenden Schritte:
Erzeugen eines charakteristischen Signals der lebenden menschlichen oder tierischen Zelle;
Erzeugen einer mechanischen Stimulation oder einer modulierten Lichtstimulation mittels eines piezoelektrischen Aktors oder Lichtaktors, der gemäß Daten des charakteristischen Signals der lebenden menschlichen oder tierischen Zelle gesteuert wird, wobei die modulierte Lichtstimulation durch Modulieren, mit dem charakteristischen Signal, einer Intensität eines Lichtstrahls erzeugt wird, der durch die lebende menschliche oder tierische Zelle absorbierbar ist;
Anwenden der mechanischen oder Lichtstimulation auf die lebende menschliche oder tierische Zelle;
wobei das charakteristische Signal durch die folgenden Vorgänge erzeugt wird:
Platzieren der extrahierten lebenden Zelle in einer Testlösung;
Erfassen einer Schwingung eines oberflächlichen Abschnitts einer äußeren Oberfläche der extrahierten lebenden Zelle;
Erzeugen eines gewandelten zeitveränderlichen Signals, das der erfassten Schwingung entspricht;
Tiefpassfiltern von Werten des gewandelten zeitveränderlichen Signals in dem Frequenzbereich unter 20 Hz, Erzeugen von Daten des charakteristischen Signals als eine tiefpassgefilterte Nachbildung der Werte des gewandelten zeitveränderlichen Signals.

2. Verfahren nach Anspruch 1, wobei das gewandelte Signal erzeugt wird durch:
Beschaffen und Installieren eines Rasterkraftmikroskops, das eine Tastspitze aufweist;
Platzieren der Tastspitze in Kontakt mit der äußeren Oberfläche der extrahierten lebenden Zelle;
Erzeugen des gewandelten Signals als ein elektrisches Signal, das für Schwingungen, die durch die Tastspitze erfasst werden, repräsentativ ist.

3. Verfahren nach Anspruch 1, wobei das gewandelte Signal erzeugt wird durch:
Beschaffen und Installieren eines Beleuchtungslasers, der konfiguriert ist, um die äußere Oberfläche der Zelle zu beleuchten;
Beschaffen und Installieren eines optischen Sensors, der konfiguriert ist, um Licht zu erfassen, das durch die äußere Oberfläche der Zelle gestreut wird;
Erzeugen des gewandelten Signals als ein elektrisches Signal, das für Intensität von Licht repräsentativ ist, das durch den optischen Sensor empfangen wird, das durch die äußere Oberfläche gestreut wird.

4. Verfahren nach einem der vorstehenden Ansprüche, wobei das charakteristische Signal als eine tiefpassgefilterte Nachbildung von Werten des gewandelten Signals in dem Frequenzbereich unter 10 Hz, vorzugsweise unter 8 Hz, erzeugt wird.

5. Vorrichtung zum Stimulieren einer lebenden menschlichen oder tierischen Zelle entweder in vivo oder in vitro, die aus einem menschlichen oder tierischen Körper entnommen wurde, umfassend:
einen gesteuerten piezoelektrischen oder Lichtaktor, der konfiguriert ist, um eine mechanische oder Lichtstimulation zu erzeugen, die auf die lebende menschliche oder tierische Zelle anzuwenden ist;
eine Mikroprozessoreinheit, die konfiguriert ist, um Daten eines charakteristischen Signals der lebenden menschlichen oder tierischen Zelle zu empfangen, das durch die folgenden Vorgänge erzeugt wird:
Platzieren der extrahierten lebenden Zelle in einer Testlösung,
Erfassen einer Schwingung eines oberflächlichen Abschnitts einer äußeren Oberfläche der extrahierten lebenden Zelle,
Erzeugen eines gewandelten zeitveränderlichen Signals, das der erfassten Schwingung entspricht,
Tiefpassfiltern von Werten des gewandelten zeitveränderlichen Signals in dem Frequenzbereich unter 20 Hz, Erzeugen von Daten des charakteristischen Signals als eine tiefpassgefilterte Nachbildung der Werte des gewandelten zeitveränderlichen Signals;
wobei die Mikroprozessoreinheit konfiguriert ist, um entsprechende Befehlssignale für den piezoelektrischen oder Lichtaktor zu erzeugen, um den piezoelektrischen oder Lichtaktor zu veranlassen, die mechanische oder Lichtstimulation zu erzeugen, die den Daten des charakteristischen Signals entspricht.

6. Vorrichtung nach Anspruch 5, ferner umfassend einen nichtflüchtigen Speicher, der die Daten des charakteristischen Signals speichert, wobei der Mikroprozessor konfiguriert ist, um die Daten des charakteristischen Signals aus dem nichtflüchtigen Speicher herunterzuladen und die Daten zu verarbeiten, wenn die Befehlssignale für den piezoelektrischen oder Lichtaktor erzeugt werden müssen.

7. Vorrichtung nach Anspruch 5 oder 6, ferner umfassend einen Strommodulator, der konfiguriert ist, um durch die Mikroprozessoreinheit gesteuert zu werden und eine Wellenform einer Steuerspannung oder eines Steuerstroms zu erzeugen, die den Daten des charakteristischen Signals entspricht,
wobei der Strommodulator wirkgekoppelt ist, um den gesteuerten piezoelektrischen oder Lichtaktor mit der Steuerspannung oder dem Steuerstrom zu steuern, um den piezoelektrischen oder Lichtaktor zu veranlassen, die mechanische oder Lichtstimulation entsprechend den Daten des charakteristischen Signals zu erzeugen.

8. Vorrichtung nach Anspruch 7, wobei:
der Aktor eine Vielzahl von Gruppen von LEDs umfasst, die durch eine jeweilige Vielzahl von elektrischen Leitungen versorgt werden, wobei die LEDs jeder Gruppe untereinander elektrisch in Reihe geschaltet und konfiguriert sind, um durch eine entsprechende elektrische Leitung der elektrischen Leitungen versorgt zu werden;
die Vorrichtung ferner einen Kanalwähler umfasst, der durch die Mikroprozessoreinheit über ein Befehlssignal gesteuert wird und mit dem Strommodulator und dem Aktor verbunden ist, konfiguriert, um von dem Strommodulator einen Strom zu empfangen und die Gruppen von LEDs über die elektrischen Leitungen in Abhängigkeit von dem Befehlssignal, das durch die Mikroprozessoreinheit erzeugt wird, selektiv zu versorgen.

9. Vorrichtung nach Anspruch 8, wobei jede Gruppe von LEDs der Vielzahl von Gruppen von LEDs des Aktors in einer entsprechenden hexagonalen Bestrahlungszone einer Leiterplatte angeordnet ist und eine Vielzahl von LEDs umfasst, die untereinander elektrisch in Reihe geschaltet und entlang eines Umfangs und in einer Mitte der entsprechenden hexagonalen Bestrahlungszone angeordnet sind.

10. Vorrichtung nach Anspruch 8, wobei jede Gruppe von LEDs der Vielzahl von Gruppen von LEDs des Aktors in einer Bestrahlungszone einer FPCB (Flexible Leiterplatte) angeordnet ist, die eine Vielzahl von elektrisch in Reihe geschalteten LEDs umfasst, wobei die LEDs räumlich nach einem gleichseitigen Dreiecksmuster platziert sind.

11. Vorrichtung nach Anspruch 8 oder 9, wobei der Aktor als ein tragbares Band geformt ist, das direkt in Kontakt mit einer Haut eines Benutzers getragen werden kann.

12. Vorrichtung nach Anspruch 8 oder 9, wobei der Aktor in Form eines Helms/einer Kappe geformt ist, der/die direkt in Kontakt mit der Kopfhaut getragen werden kann, oder der Aktor in Form eines Aufbisses geformt ist, der in dem Mund eines Benutzers in Kontakt mit der Zahnfleischschleimhaut und/oder den Zähnen des Benutzers zu platzieren ist.

## Revendications

1. Procédé de stimulation d'une cellule vivante humaine ou animale in vivo ou in vitro extraite d'un corps humain ou animal, comprenant les étapes suivantes consistant à :
générer un signal caractéristique de la cellule vivante humaine ou animale ;
générer une stimulation mécanique ou une stimulation par lumière modulée au moyen d'un actionneur piézoélectrique ou de lumière commandé selon des données du signal caractéristique de ladite cellule vivante humaine ou animale, dans lequel ladite stimulation par lumière modulée est générée en modulant, avec ledit signal caractéristique, une intensité d'un faisceau de lumière pouvant être absorbé par ladite cellule vivante humaine ou animale ;
appliquer ladite stimulation mécanique ou par lumière à ladite cellule vivante humaine ou animale ;
dans lequel ledit signal caractéristique est généré par le biais des opérations suivantes :
la disposition de la cellule vivante extraite dans une solution d'essai ;
la détection d'une vibration d'une partie superficielle d'une surface externe de ladite cellule vivante extraite ;
la génération d'un signal transduit variant dans le temps correspondant à ladite vibration détectée ;
le filtrage passe-bas de valeurs dudit signal transduit variant dans le temps dans la gamme de fréquences inférieure à 20 Hz, la génération de données dudit signal caractéristique sous la forme d'une réplique filtrée passe-bas des valeurs dudit signal transduit variant dans le temps.

2. Procédé selon la revendication 1, dans lequel ledit signal transduit est généré par :
acquisition et installation d'un microscope à force atomique ayant une pointe de détection ;
mise de la pointe de détection en contact avec la surface externe de la cellule vivante extraite ;
génération dudit signal transduit sous la forme d'un signal électrique représentatif de vibrations détectées par ladite pointe de détection.

3. Procédé selon la revendication 1, dans lequel ledit signal transduit est généré par :
acquisition et installation d'un laser d'éclairage configuré pour éclairer la surface externe de la cellule ;
acquisition et installation d'un capteur optique configuré pour détecter de la lumière diffusée par la surface externe de la cellule ;
génération dudit signal transduit sous la forme d'un signal électrique représentatif d'une intensité de lumière, reçue par le capteur optique, qui a été diffusée par ladite surface externe.

4. Procédé selon l'une des revendications précédentes, dans lequel ledit signal caractéristique est généré sous la forme d'une réplique filtrée passe-bas de valeurs dudit signal transduit dans la gamme de fréquences inférieure à 10 Hz, de préférence inférieure à 8 Hz.

5. Appareil de stimulation d'une cellule vivante humaine ou animale in vivo ou in vitro extraite d'un corps humain ou animal, comprenant :
un actionneur piézoélectrique ou de lumière commandé, configuré pour générer une stimulation mécanique ou par lumière à appliquer à la cellule vivante humaine ou animale ;
une unité de microprocesseur, configurée pour recevoir des données d'un signal caractéristique de ladite cellule vivante humaine ou animale généré par le biais des opérations suivantes :
la disposition de la cellule vivante extraite dans une solution d'essai,
la détection d'une vibration d'une partie superficielle d'une surface externe de ladite cellule vivante extraite,
la génération d'un signal transduit variant dans le temps correspondant à ladite vibration détectée,
le filtrage passe-bas de valeurs dudit signal transduit variant dans le temps dans la gamme de fréquences inférieure à 20 Hz, la génération de données dudit signal caractéristique sous la forme d'une réplique filtrée passe-bas des valeurs dudit signal transduit variant dans le temps ;
ladite unité de microprocesseur étant configurée pour générer des signaux de commande correspondants pour l'actionneur piézoélectrique ou de lumière afin d'amener l'actionneur piézoélectrique ou de lumière à générer la stimulation mécanique ou par lumière correspondant aux données du signal caractéristique.

6. Appareil selon la revendication 5, comprenant en outre une mémoire non volatile stockant lesdites données du signal caractéristique, dans lequel ledit microprocesseur est configuré pour télécharger lesdites données du signal caractéristique à partir de la mémoire non volatile et pour traiter les données lorsque les signaux de commande pour l'actionneur piézoélectrique ou de lumière doivent être générés.

7. Appareil selon la revendication 5 ou 6, comprenant en outre un modulateur de courant configuré pour être commandé par l'unité de microprocesseur et pour générer une forme d'onde d'une tension de commande ou d'un courant de commande correspondant auxdites données du signal caractéristique,
dans lequel ledit modulateur de courant est couplé fonctionnellement pour commander ledit actionneur piézoélectrique ou de lumière commandé avec ladite tension de commande ou ledit courant de commande afin d'amener l'actionneur piézoélectrique ou de lumière à générer la stimulation mécanique ou par lumière correspondant aux données du signal caractéristique.

8. Appareil selon la revendication 7, dans lequel :
ledit actionneur comprend une pluralité de groupes de DEL alimentés par une pluralité respective de lignes électriques, les DEL de chaque groupe étant connectées électriquement en série parmi elles et configurées pour être alimentées par le biais d'une ligne électrique correspondante desdites lignes électriques ;
l'appareil comprend en outre un sélecteur de canal commandé par l'unité de microprocesseur par le biais d'un signal de commande et connecté au modulateur de courant et audit actionneur, configuré pour recevoir à partir dudit modulateur de courant un courant et pour alimenter sélectivement les groupes de DEL par le biais desdites lignes électriques en fonction dudit signal de commande généré par l'unité de microprocesseur.

9. Appareil selon la revendication 8, dans lequel chaque groupe de DEL de ladite pluralité de groupes de DEL de l'actionneur est attribué dans une zone d'irradiation hexagonale correspondante d'une carte de circuits imprimés et comprend une pluralité de DEL connectées électriquement en série parmi celles-ci disposées le long d'un périmètre et au niveau d'un centre de la zone d'irradiation hexagonale correspondante.

10. Appareil selon la revendication 8, dans lequel chaque groupe de DEL de ladite pluralité de groupes de DEL de l'actionneur est attribué dans une zone d'irradiation de FPCB (carte de circuits imprimés flexible), qui comprend une pluralité de DEL connectées électriquement en série, dans lequel les DEL sont disposées dans l'espace suivant un motif triangulaire équilatéral.

11. Appareil selon la revendication 8 ou 9, dans lequel ledit actionneur a la forme d'une bande portable qui peut être portée directement en contact avec la peau d'un utilisateur.

12. Appareil selon la revendication 8 ou 9, dans lequel ledit actionneur se présente sous la forme d'un casque/capuchon qui peut être porté directement en contact avec le cuir chevelu, ou ledit actionneur se présente sous la forme d'un morceau à être disposé à l'intérieur de la bouche d'un utilisateur en contact avec la muqueuse gingivale et/ou les dents de l'utilisateur.
